Europäisches Patentamt

European Patent Office

Office européen des brevets

(11) Publication number: **0 424 686 A1**

# EUROPEAN PATENT APPLICATION

(21) Application number: 90118667.6

(51) Int. Cl.5: **A61B 17/32, A61F 9/00**

(22) Date of filing: **28.09.90**

(30) Priority: **27.10.89 US 428232**

(43) Date of publication of application:
**02.05.91 Bulletin 91/18**

(84) Designated Contracting States:
**DE FR GB IT**

(71) Applicant: **STORZ INSTRUMENT COMPANY**
**3365 Tree Court Industrial Boulevard**
**St Louis Missouri 63122(US)**

(72) Inventor: **Appelbaum, Peter F.**
**5072 Oleatha, Apt. B.**
**St. Louis, Missouri 63139(US)**
Inventor: **Ameiss, Michael S.**
**7155 Oak Stream**
**O'Fallon, Missouri 63052(US)**
Inventor: **McFerran, Stanley C.**
**3222 East Romaine**
**Imperial, Missouri 63052(US)**
Inventor: **Painter, John A.**
**219 Maple Point Drive**
**St. Charles, Missouri 63303(US)**

(74) Representative: **Wächtershäuser, Günter, Dr.**
**Tal 29**
**W-8000 München 2(DE)**

(54) Control system for ophthalmic surgical instruments.

(57) A microprocessor-based electronic control system for controlling microsurgical instruments includes a display screen (50) and a plurality of operator input switches (52) which support various enhanced user-friendly functions. The operator interface provides special utility display screens for allowing a user to change various default settings for operating parameters, the screen seen upon power up mode, and the language set used for the screens. The control system includes a main control console, and a processor which is programmed to display a variety of screens in a predetermined sequence or tree structure to facilitate user changes to the system. The control system also includes set-up screens for adjusting monitoring and warning sounds in tone and volume. Also, the control system provides a user-selectable rate of change in aspiration levels to permit users to obtain a speed of response in aspiration which they are accustomed or most comfortable with.

FIG.IA

## CONTROL SYSTEM FOR OPHTHALMIC SURGICAL INSTRUMENTS

### FIELD OF THE INVENTION

The present invention relates in general to ophthalmic microsurgical control systems having a control console provided with a two-dimensional display for presenting screens of information in any desired sequence, and in particular to enhanced operator interfaces for ophthalmic microsurgical control systems which allow a user to personally configure default parameter values and operating settings used during ophthalmic surgical procedures.

### BACKGROUND OF THE INVENTION

In the ophthalmic microsurgical art, there are a number of different arrangements known for providing operator interfaces with pneumatic and electronic control consoles used to power and operate micro surgical instruments such as phacoemulsification probes, irrigation needles, air-exchange needles, vitrectomy probes, microsurgical scalpels used in controlled anterior capsulotomy (CAC) procedures, bipolar coagulation electrodes, aspiration needles, and the like. In the past, separate control cabinets were provided for individual pieces of equipment required to power and operate one particular instrument, for example, a guillotine-type vitrectomy probe, which requires aspiration and a pulsating pneumatic signal to drive its internal spring-biased piston. Such a control cabinet is typically provided with an adjustment knob for regulating air pressure, another knob for regulating the frequency of the pulsating pneumatic signal, and oftentimes an LED read-out of the vacuum level used for aspiration, and another LED display in cycles per minute ("cpm") for displaying the cut rate or frequency of the pneumatic driving signal. As another example, it is known to provide a self-contained control module for producing electrical energy at ultrasonic frequencies for driving a phacoemulsification probe. This control module would typically include one or more adjustment knobs for changing the level of electrical input power to the probe, the duty cycle of the electrical signal, and a plurality of LED displays for providing a read-out of pertinent information such as average power level and an elapsed time value indicating the total time ultrasonic energy has been utilized during the surgical procedure. Such phaco probes also normally require aspiration to suction away disintegrated fragments and other debris created by use of the phaco probe, and a supply of irrigation to help wash away such disintegrated fragments.

Thus, in order to have a complete ophthalmic surgical system capable of performing all operations, a hospital or clinic purchased several control modules, each in their own enclosure, which could be used separately or simultaneously, depending upon the requirements of the particular surgical procedure. However, each control cabinet had its own operator interface, with LED displays, dials, knobs and buttons as described above. Each also had its own separate connector or port for plugging in the appropriate instrument cable, tubing line or other needed connector. In this environment, each separate surgical piece of equipment was operating autonomously, at least in a physical sense.

Such pieces of individually designed equipment do not provide a common operator interface for selecting all of these functions. One consequence of this practice was a multiplicity of footpedals, one for each separately controlled instrument. This situation was not entirely satisfactory to surgeons, so a more integrated approach to interfacing various pieces of control equipment evolved. A number of newer systems now provide a footpedal with a plurality of switches mounted thereon so that more than one function can be controlled via a single footpedal.

A few years ago, the assignee of the present invention, namely Storz Instrument Company of St. Louis, Missouri (hereinafter "Storz"), introduced to the market a fully integrated control console for ophthalmic surgery for use in performing almost all types of ophthalmic surgical procedures. This integrated control system and console is sold under the trademark "DAISY" and has enjoyed considerable commercial success. It supported a wide variety of microsurgical instruments. One of the unique features of the DAISY control console is its use of a CRT display with two columns of five membrane-type switches adjacent either vertical side of the display screen and a horizontal row of four endless digital potentiometers adjacent the bottom of the display screen. A slot for an aspirant collection cassette was provided in the lower-front corner of the console, and a horizontal row of instrument connector ports was provided below the row of potentiometers, allowing a variety of microsurgical instruments to be plugged in. A footpedal assembly for use by the surgeon conducting the operation was also provided. The DAISY console also included a pneumatic system for producing aspiration and pulsating pneumatic signals for driving various instruments such as guillotine cutters used in vitrectomies and microscissors used for vitreoretinal operations, and electrical systems for bipolar cautery and phacoemulsification.

A number of ophthalmic surgeons prefer to perform certain kinds of ophthalmic surgical procedures using predetermined settings for operating parameters such as maximum aspiration level, maximum cut rate, maximum IOP pressure, minimum phaco power and the like. In an effort to meet this need, the DAISY console provides a factory-programmable memory key containing nonvolatile integrated memory circuits. This memory key is insertable into the DAISY console and will, upon initialization of the console, cause the original factory-established default settings for various parameters to be replaced with those preferred by a particular surgeon which have been pre-programmed into the memory key. This type of function is described in aforementioned application Serial No. 06/928,170 entitled "Control System For Ophthalmic Surgical Instruments." However, the DAISY console did not otherwise allow a particular user to alter the factory-established default settings for various parameters. Thus, surgeons could not easily experiment with which default settings were best for them, and could not change such default settings to suit evolving surgical procedures without having a Storz service representative re-program the memory key.

Further, a number of surgeons using the DAISY console find that its operating characteristics are somewhat different than the characteristics of other ophthalmic microsurgical systems they may have used in the past. Perhaps the most notable example is the rate of change of aspiration. In the DAISY console, the aspiration is produced by use of an air-to-vacuum converter, namely a venturi, which produces a very rapid response in comparison to other techniques for producing aspiration such by use of a peristaltic pump or a mechanical pump with a vacuum accumulator. Also, posterior surgeons often prefer fast aspiration response, whereas many anterior surgeons prefer a considerably slower response.

Another problem encountered in an ophthalmic surgery using different pieces of equipment is that the characteristic monitoring sounds or warning sounds produced by ophthalmic control equipment varies among the equipment from different manufacturers. Further, some surgeons in a given hospital may wish to have no such sounds, while other surgeons using the same equipment at the same hospital prefer to have sounds.

In light of the foregoing desires of the ophthalmic surgical community, it is a primary object of the present invention to make it possible for the user of ophthalmic surgical equipment to adjust and store custom default settings and operating parameters via the main console. It is a related object of the present invention to allow the user to customize the screens to suit his or her specific needs when operating the equipment.

It is another primary object of the present invention to provide programmable audio generation means to produce different sounds for monitoring and/or warning purposes, and to allow such sounds to be adjusted by the user to his or her preferences.

It is still another object of the present invention to provide a mechanism for allowing the rate of change of aspiration levels to be adjusted at the control console by the user whenever the user desires to do so.

## SUMMARY OF THE INVENTION

In light of the foregoing in needs and desires, there is provided according to a first aspect of the present invention, an ophthalmic microsurgical control system for controlling a plurality of micro surgical instruments which has an improved operator interface. The control system comprises a main processor, display means, a plurality of operator-actuatable input switches. The control system further comprises means for generating a plurality of set-up menus on the display means, each of the menus listing one or more parameters which may be adjusted; and means for adjusting at least one of the parameters on at least the plurality of available set-up menus. These set-up menus may be used to adjust audio tones, the volume at which the audio tones are produced and by assigning certain tones to various surgical functions which may be in use simultaneously, determining how such tones are provided in combination.

Another one of the set-up menus provides means for controlling the aspiration function used in connection with at least one of the surgical instruments, and means are also provided for adjusting the rate at which an aspiration vacuum level is allowed to change. In the preferred embodiment of the present invention, six aspiration rate change selections are provided.

According to a second aspect of the present invention there is provided an ophthalmic microsurgical control console for operating a plurality of microsurgical instruments. The control console comprises: display means for providing a plurality of user-selectable menus to be visually presented; microprocessor means for producing set-up menus arranged in a predetermined organizational structure; operator input means for selecting any one of the set-up menus; and non-volatile memory means for storing user selected parameter values. The control console may further comprise means for adjusting default parameters associated with the plurality of anterior segment surgical procedures; and means for adjusting default parameters associated with posterior segment surgical procedures.

These and other aspects, advantages and features of the present invention will be better understood upon studying the following detailed description and accompanying figures in conjunction with the appended claims.

BRIEF DESCRIPTION OF THE DRAWINGS

The drawings form an integral part of the description of the preferred embodiments and are to be read in conjunction therewith. Like reference numerals designate the same or similar components or features in the various Figures, where:

Figures 1A and 1B are front and back perspective views of an ophthalmic microsurgical control console which utilizes the enhanced operator interfaces and other aspects of the present invention;

Figure 2 is a front view of the Figure 1 control console showing the lay-out of the CRT visual display, control buttons or keys, surgical instrument connection ports and the like;

Figure 3 is a plan view of the footswitch assembly usable with the present invention;

Figure 4A is a simplified block diagram of the microprocessor-based electronic control system of the Figure 1 control console showing how information is passed electronically between the microprocessor and the various boards and devices within the over-all surgical system;

Figure 4B is a detailed block diagram of the operation of the CAC/bipolar board and equipment of the Figure 1 control console;

Figure 4C is a detailed block diagram of the calibration and drive system for a phaco probe;

Figure 4D is a detailed block diagram of the illumination lamp control circuitry and electrical hardware associated therewith;

Figure 4E is a detailed block diagram of the pneumatics control and cassette control circuitry and related electrical equipment found in the pneumatic system and cassette system of the Figure 1 console;

Figure 4F is a detailed block diagram of the I/O expansion board shown in Figure A and the electrical equipment interfaced therewith; and

Figure 5 is a tree diagram showing the sequence and organization of the various menus used in the improved Figure 1 ophthalmic microsurgical system of the present invention;

Figures 6 through 18 show various menus or display screens projected on to display 50 of the Figure 1 console, where:

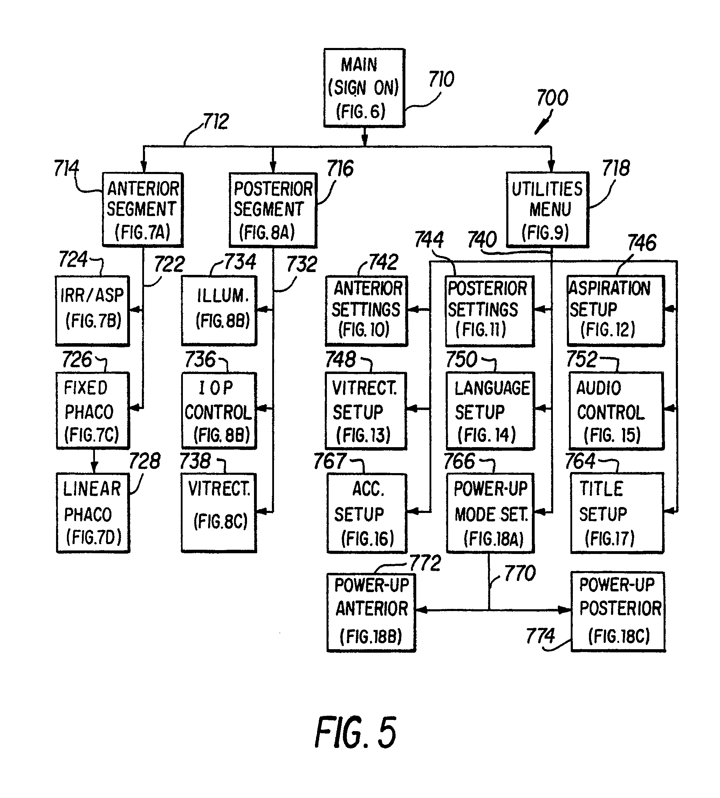

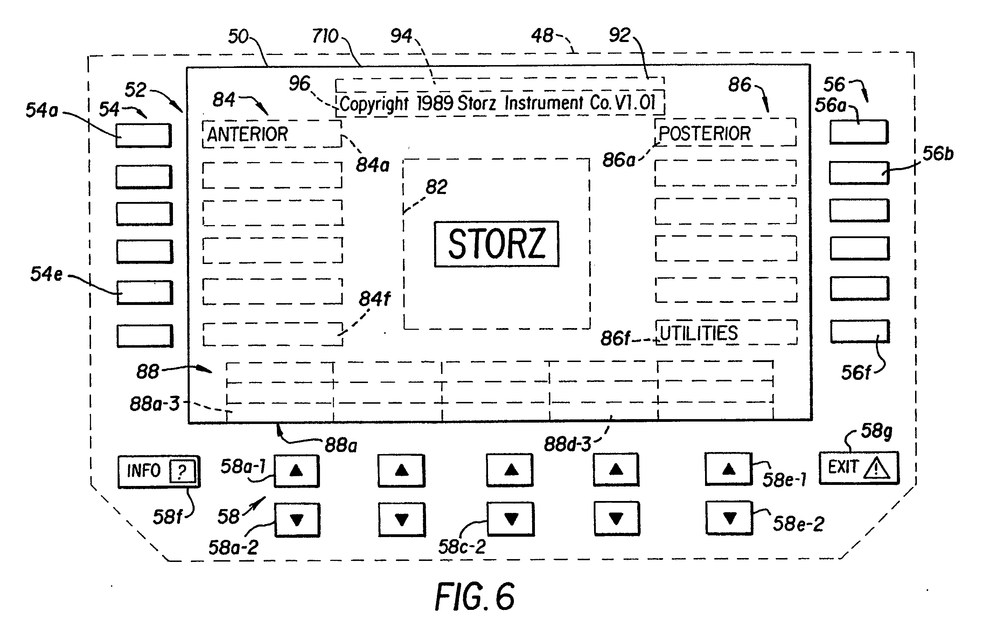

Figure 6 is a main or sign-on screen;

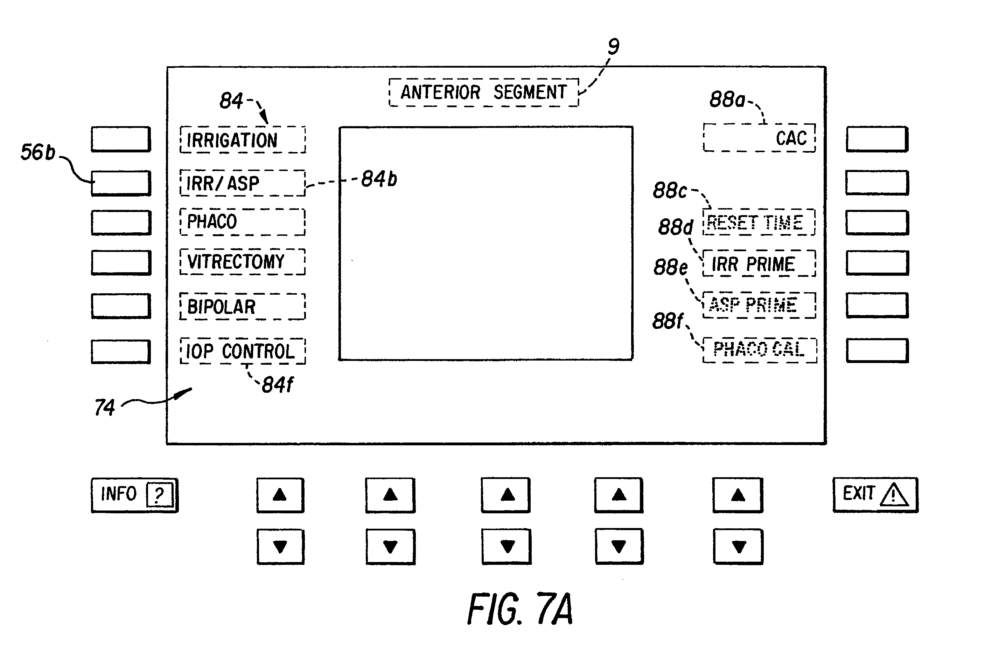

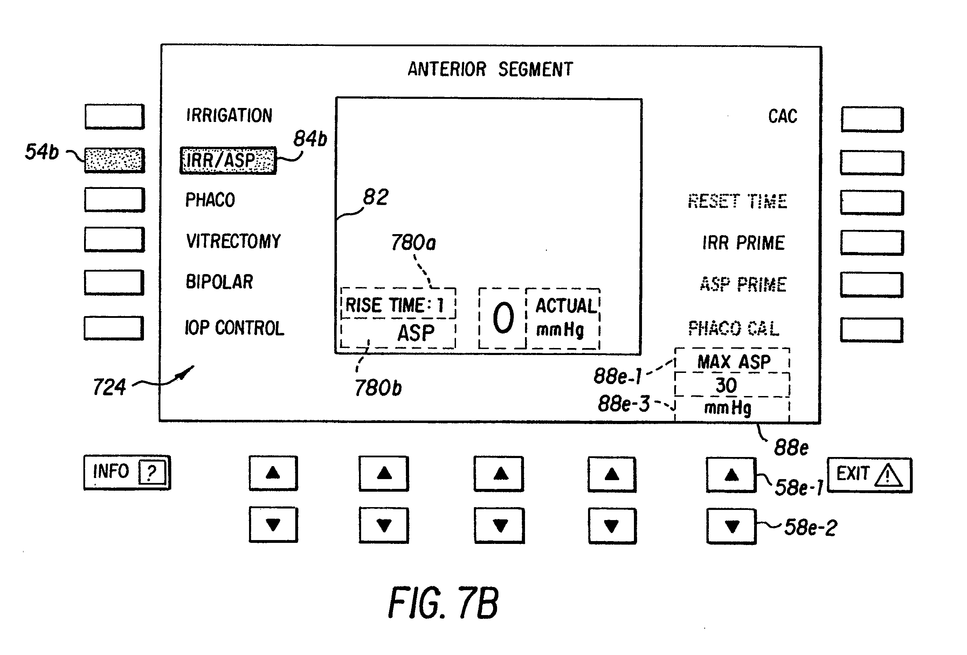

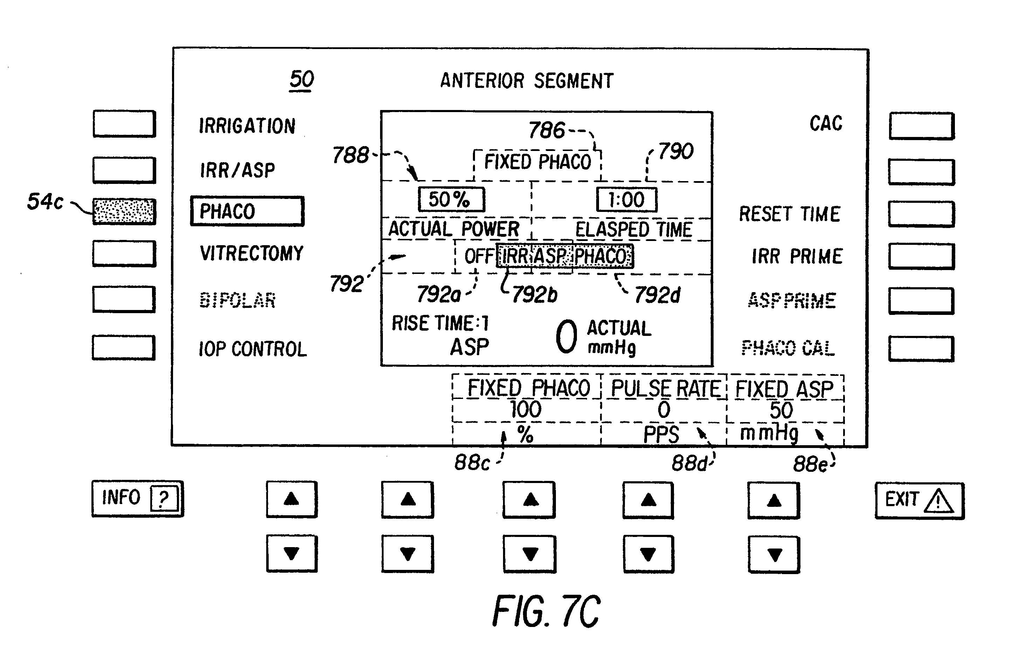

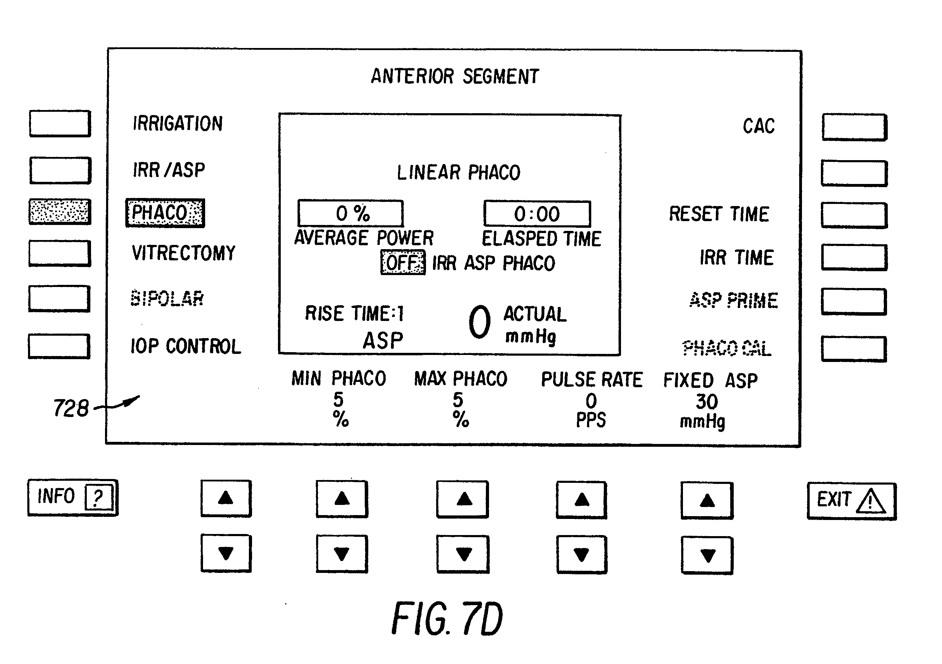

Figure 7A is the main anterior segment screen through which are accessed the display screens shown in Figures 7B, 7C and 7D which are respectively devoted to the irrigation/aspiration mode, the fixed phaco mode, and the linear phaco mode;

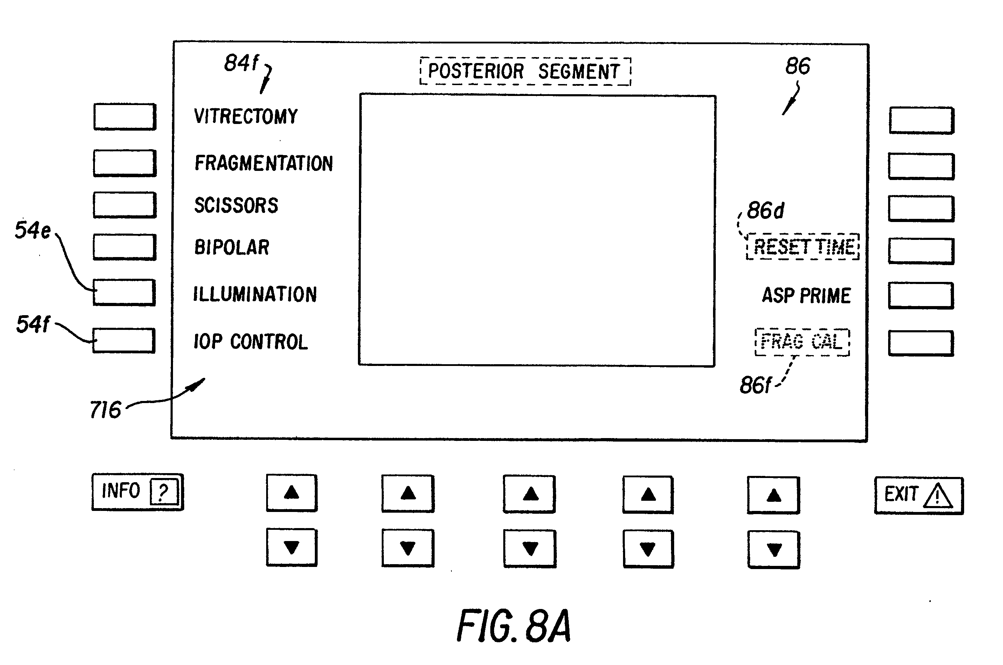

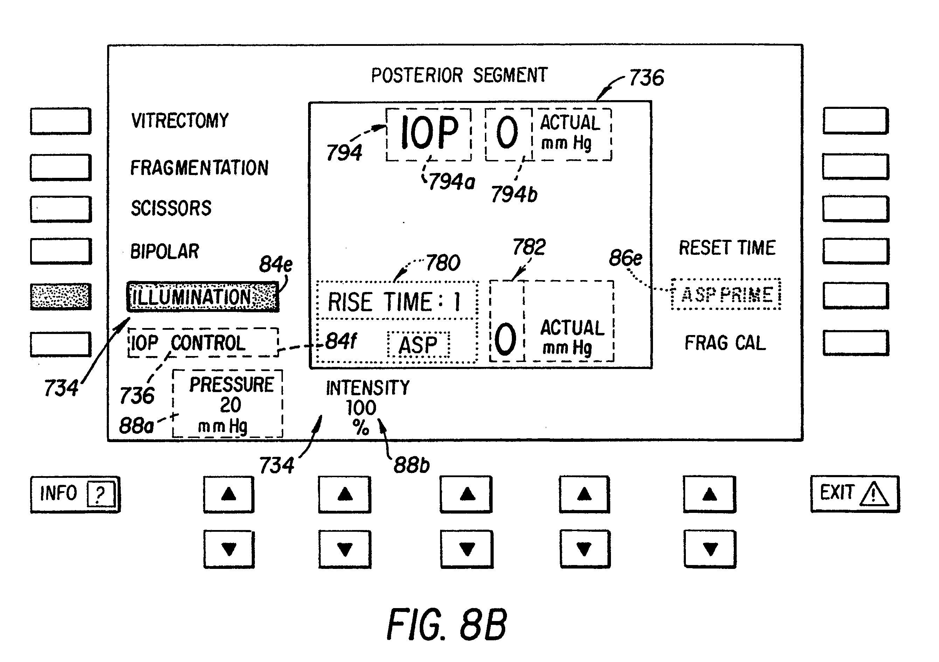

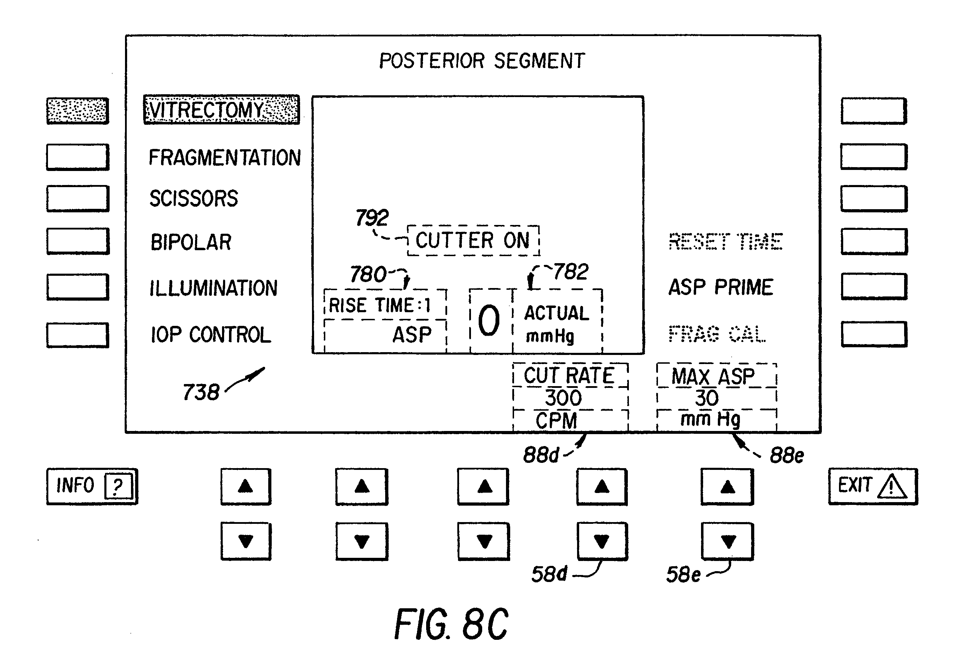

Figure 8A is the main posterior segment screen, through which are accessed the display screens of Figures 8B and 8C which are respectively used for the illumination and I/O control modes and the vitrectomy mode;

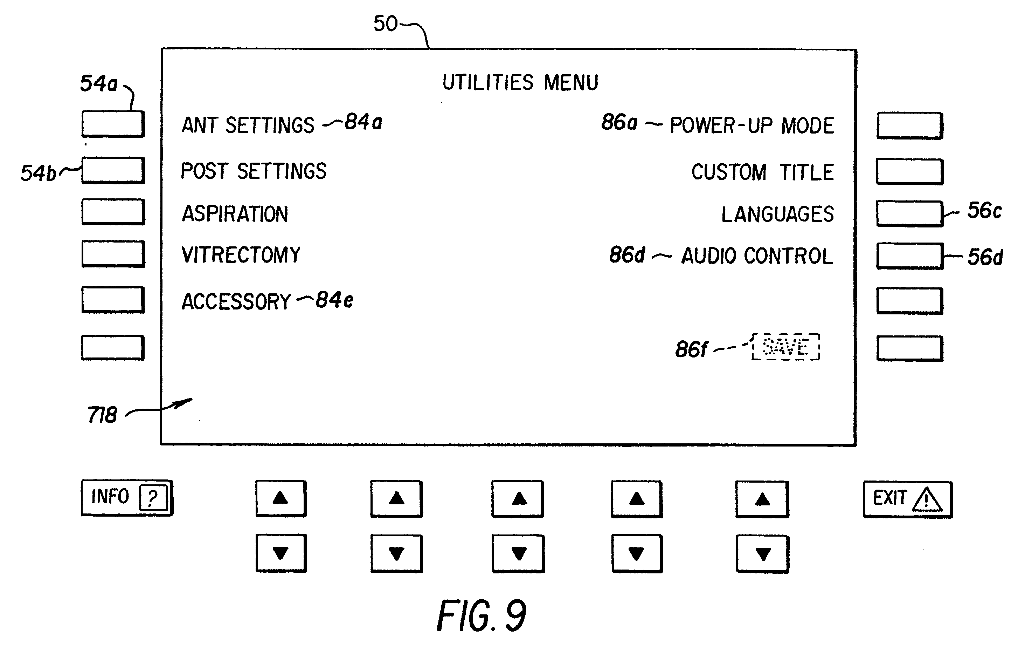

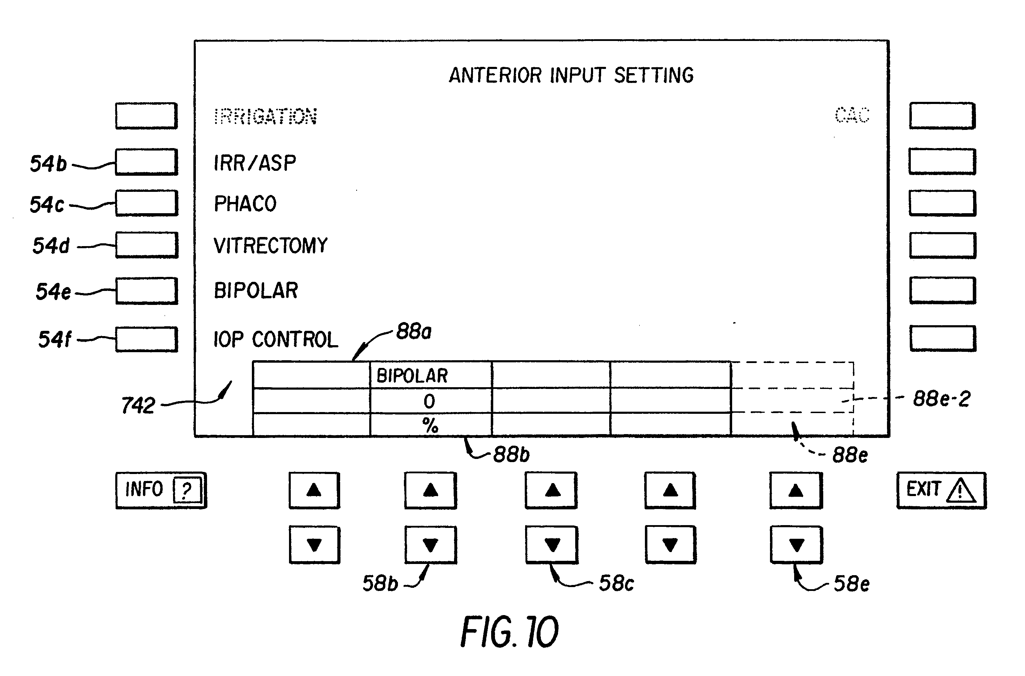

Figure 9 is the main utilities menu, through which are accessed the various utilities screens shown in Figures 10-18;

Figure 10 is the anterior input settings screen;

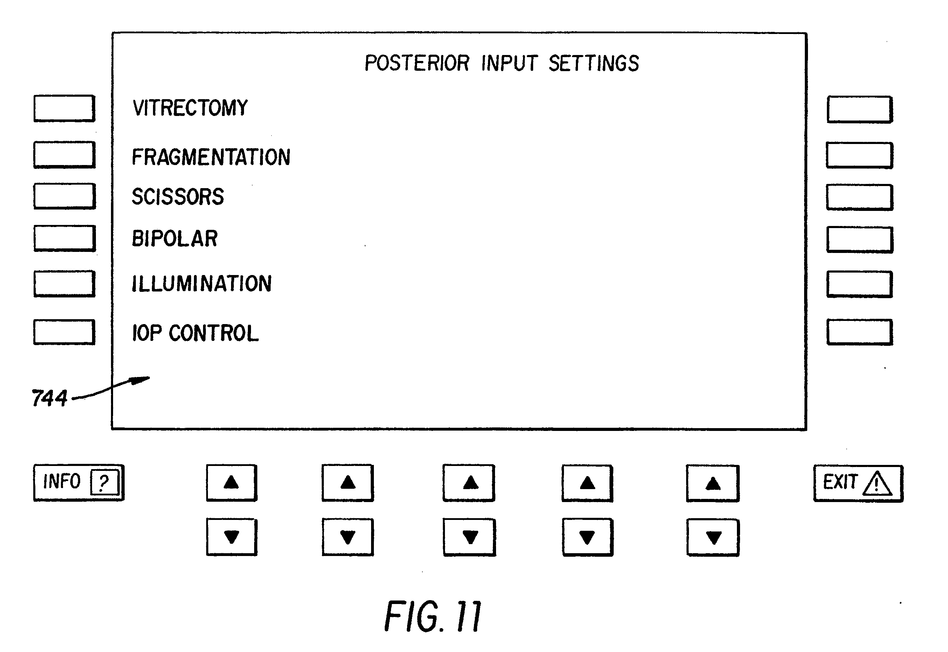

Figure 11 is the posterior input setting screen;

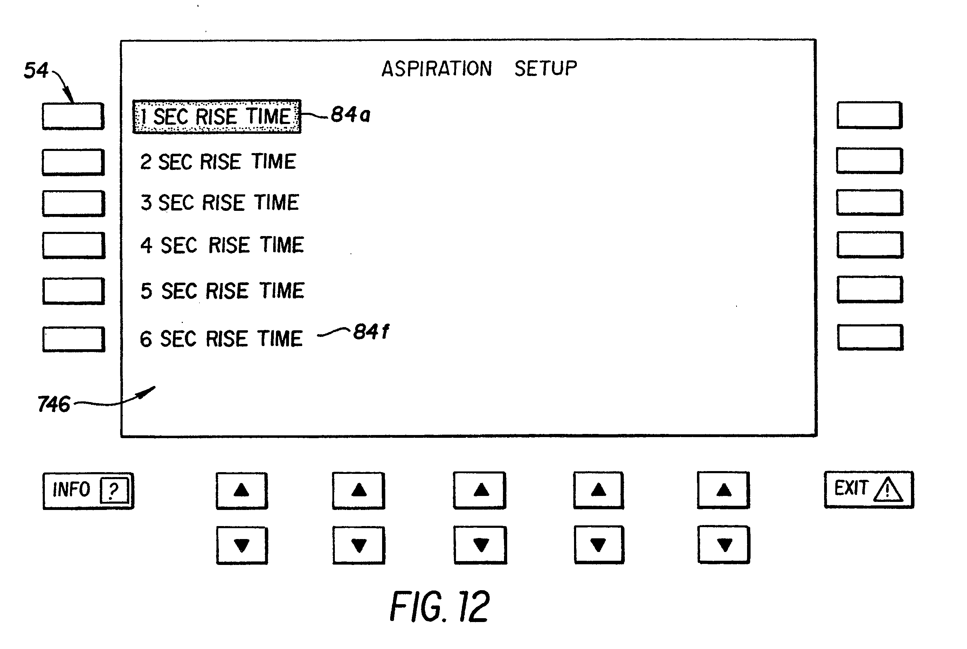

Figure 12 is the aspiration rise time setup screen;

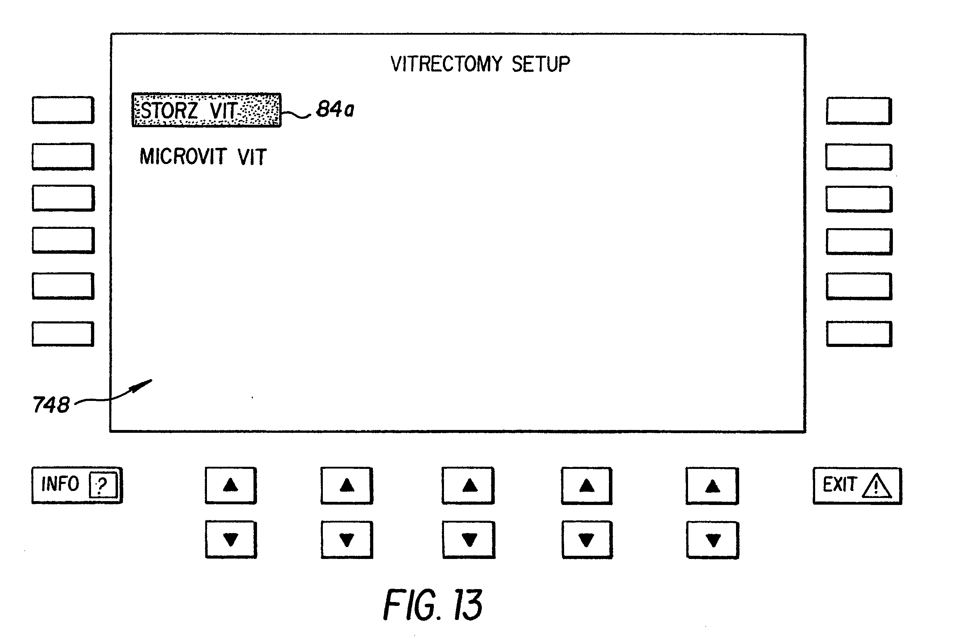

Figure 13 is the vitrectomy setup screen;

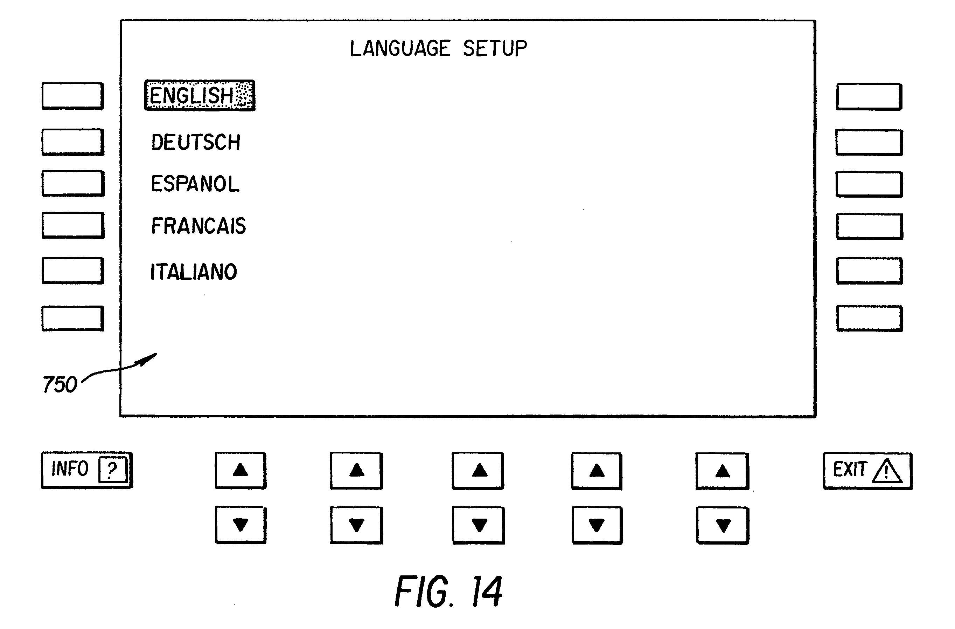

Figure 14 is the language setup screen;

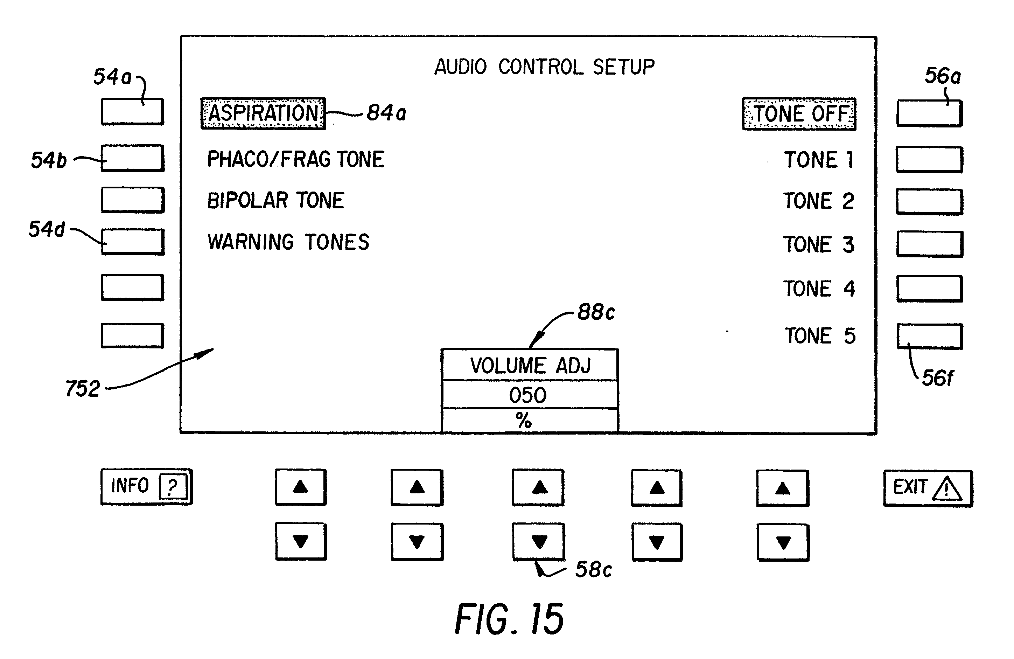

Figure 15 is the audio control setup screen;

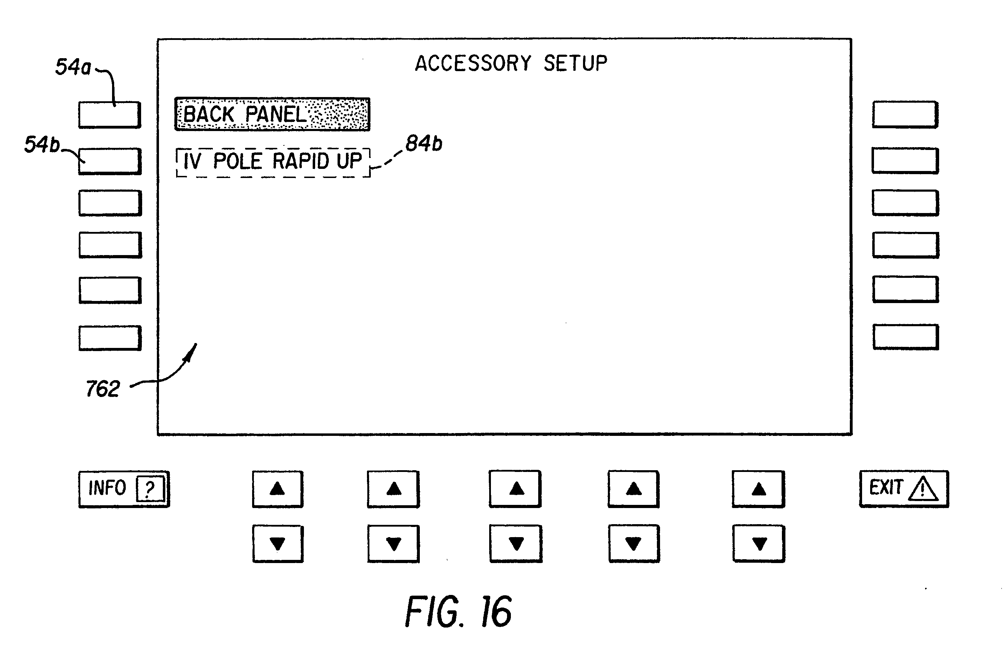

Figure 16 is the accessory setup screen;

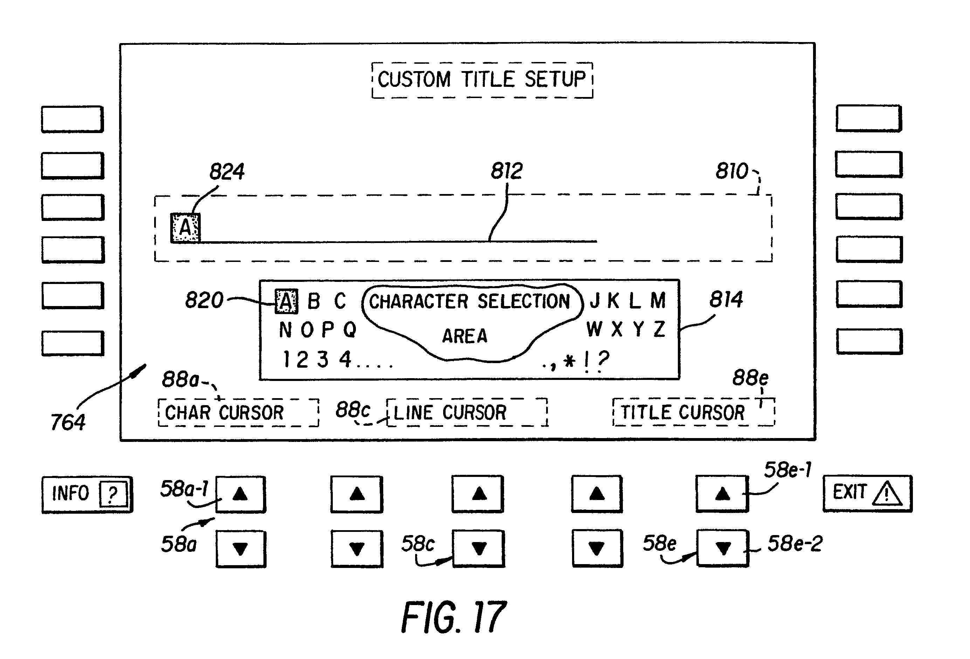

Figure 17 is the custom title setup screen; and

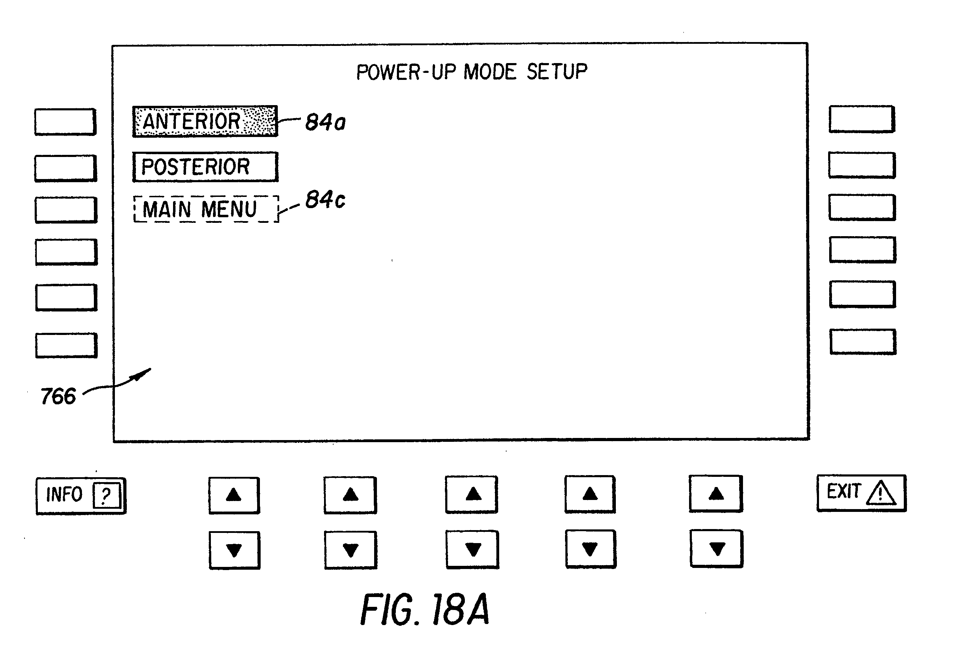

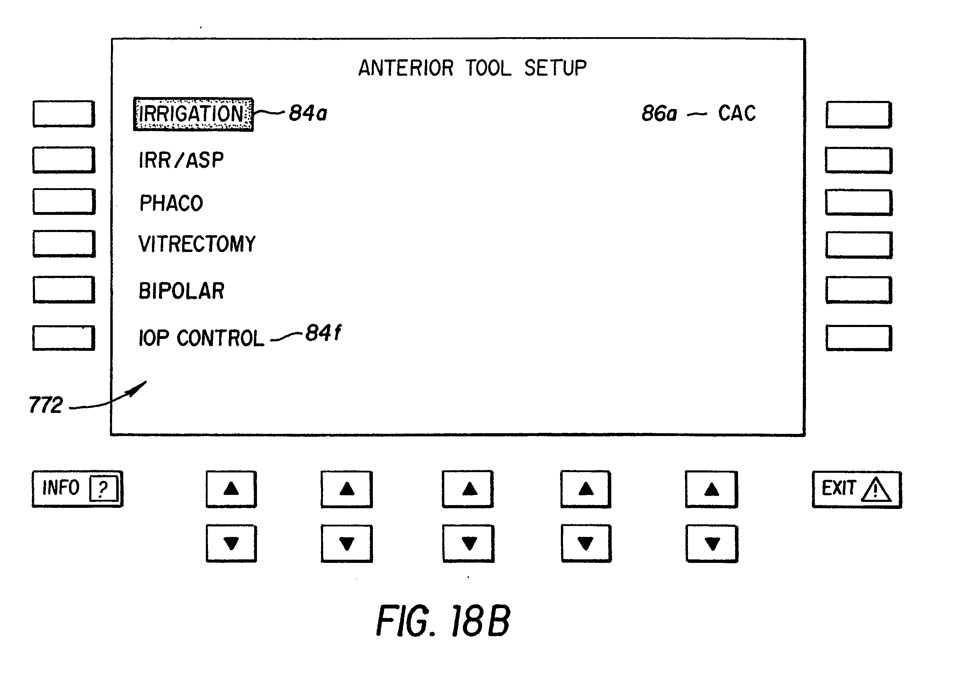

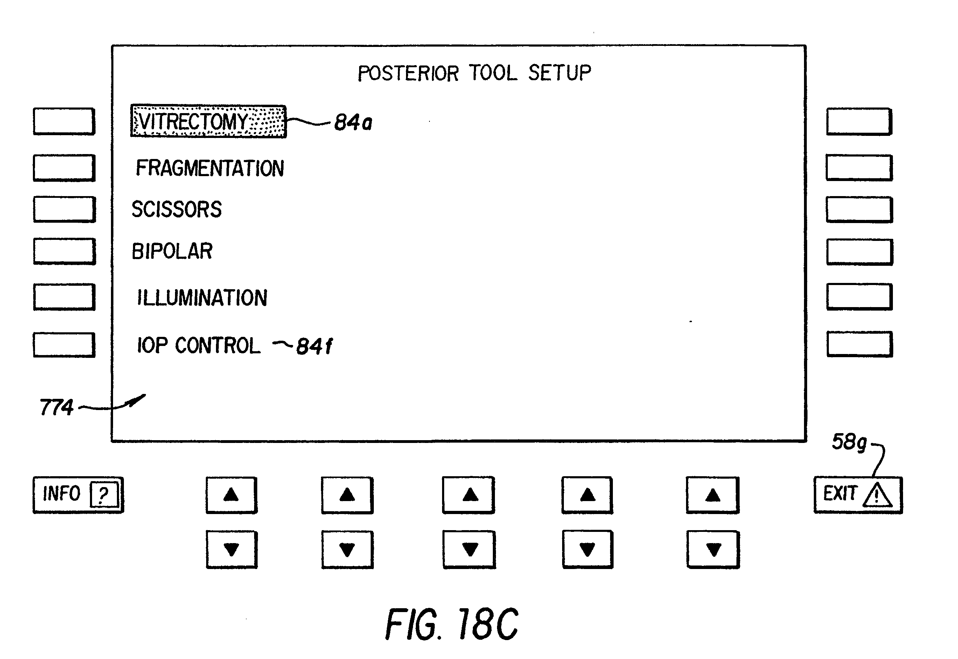

Figure 18A is the power-up mode setup screen, through which are accessed the display screens shown in Figures 18B and 18C which are respectively used for anterior tool setup and posterior tool setup.

DETAILED DESCRIPTION OF THE PREFERRED EMBODIMENTS

I. General

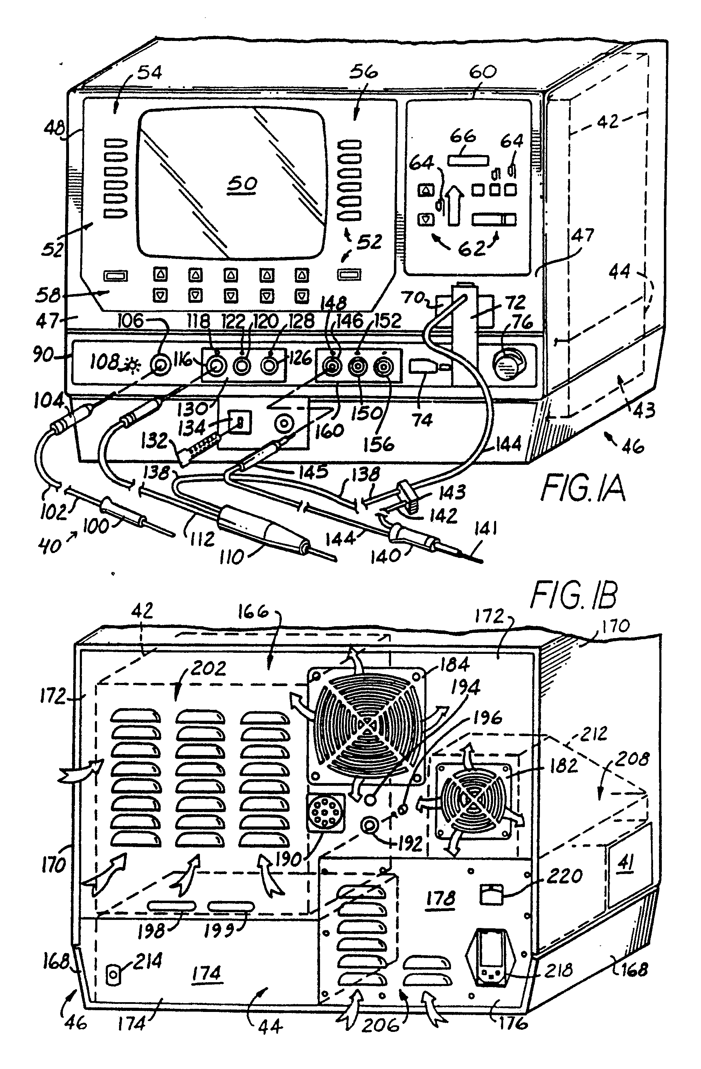

A. Front of Control Console (Fig. 1A)

1. Display & Keys Of Primary Panel

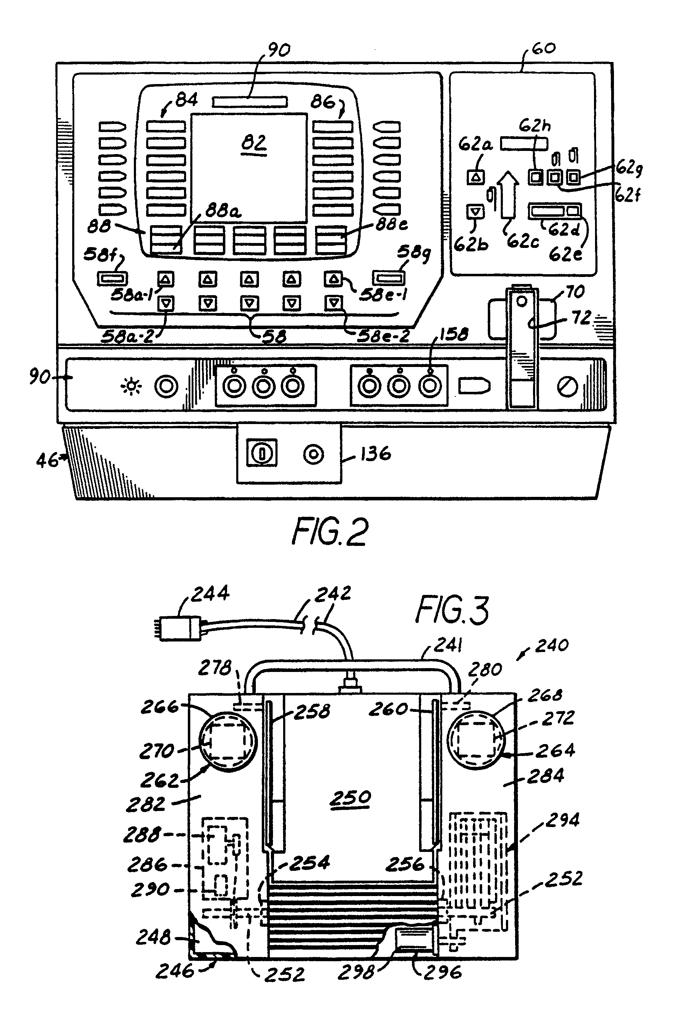

Figures 1A, 1B and 2 show a microsurgical control system 40 provided with an illumination lamp drawer 41, an electronic control system housed in part in a nine-board electronic card rack 42, and a pneumatic control system 43 housed primarily in a pneumatic drawer module 44, and other modules which will be described later. The control system 40 includes a system console 46 which has an upwardly and slightly inwardly-sloping front surface 47 with a primary front panel 48. On the front panel 48 is an electronic display screen 50, a plurality of pushbuttons or touch sensitive pads 52 organized in two groups 54 and 56 along the left and right sides of the display screen 50, and a third group 58 along the bottom of the display screen 50. Additionally, there is a secondary front panel 60 located to the right of front panel 48 which has additional pushbuttons or pads 62, indicator lights 64 and information readout 66. The console 46 also includes a slot 70 for a conventional Storz aspirant collection cassette 72, a cassette eject button 74 and an irrigation pinch valve assembly 76.

The electronic display screen 50 is controlled by a microcomputer within the console 46 to provide several different menus or messages which instruct the operator as to the function of the pushbuttons 52 through 62. The operation of the display screen 50 in combination with the buttons 52-62 may be best understood by looking at the enlarged view in Figure 2. The display screen 50 is shown there as being conceptually divided into central display screen region 82, left-side display region 84, right-side display region 86, bottom display region 88 and a top display region 90. The side regions 84 and 86 each consist of six horizontal fields stacked one above the other and positioned to correspond to the locations of buttons in button groups 54 and 56. By virtue of the adjacent location of the top button of button group 54 and the top field of region 84, for example, a message in the upper left-hand corner of the screen 50, i.e., in this top field, is readily understood by the operator as referring to the upper leftmost button. The other buttons and fields are similarly paired. This arrangement allows the indicated function of each of the buttons 54 or 56 to be readily changed by simply just changing the legend displayed in its adjacent field. In a similar manner, each pair of buttons, such as buttons 58a-1 and 58a-2, is associated with one of the three-part fields of bottom region 88, such as region 88a. In general, the upper row of buttons, i.e., buttons 58a-1 through 58e-1 are used to increment a setting or parameter displayed in the corresponding region 88a-88e of screen 50 directly above, while the buttons in the lower row, i.e., buttons 58a-2 through 58e-2, are used to decrement such displayed settings or parameters. The use of an electronic display screen also permits the legends for buttons 52, 54 and 56 to be labeled in virtually any language. Button 58f is used to bring up an information screen on display 50 to assist the operator, such as by further explaining functions associated with choices on the display menu. Button 58g is used to return to an earlier menu screen in a chain of related menus or other screens.

2. Surgical Instrument Connector Panel

The micro surgical control system 40 is capable of operating a number of different microsurgical instruments. To provide for this functionality, there is a row of different types of connector receptacles on surgical instrument connector panel 90 which permits various instruments to be plugged in or otherwise controlled by the control system 40 as may be seen in Figures 1A and 2, indicator lights are provided adjacent to or above each of the connector receptacles for indicating when the connector is activated or functional.

a. Illumination Instrument

Figure 1A shows a fiber-optic illumination instrument 100 coupled to console 46 via fiber-optic cable 102 which extends out of male illumination connector plug 104 designed for insertion into illumination connector receptacle 106. Indicator lamp 108 is illuminated whenever the fiber optic illumination (FOL) lamp inside console 46 is lit.

b. Electrically Powered Instruments

Phaco fragmentation handpiece 110 is a conventional piezoelectric device for disintegrating hard objects such as intraocular cataractous material utilizing ultrasonic ("US") energy transmitted to its needle 112. Electrical power pulsating at US frequency is provided to handpiece 110 via power cable 112 attached to phaco connector plug 114, which is designed to be inserted into phaco female connector 116. Light 118 indicates when US frequency electrical power is being delivered to 116. Female connector 120 is designed to receive a male connector plug 120 for powering a conventional bipolar coagulator handpiece. Indicator light 122 indicates when this connector 120 is operational. Female connector 126 is used for receiving a male connector plug (not shown) of a conventional CAC handpiece. (CAC stands for "controlled anterior capsulotomy.") Indicator 128 illuminates when the CAC function is activated. Thus it will be seen that the three connectors 116, 120 and 126 grouped together on rectangular plate 130 all relate to electrically powered surgical functions.

c. Fluid-Powered Instruments

Certain microsurgical instruments are actuated or controlled by fluid pressure (either positive pressure or negative pressure, or both). The phaco fragmentation instrument 110, for example, utilizes aspiration through hollow flexible plastic tubing 138 to remove disintegrated materials, which are collected along with aspirant in the cassette 72.

Vitrectomy probe 140 includes a hollow needle 141 having an inner tube which reciprocates to cut intraocular material sucked in a small hole near the tip of the needle. The inner tube (not shown) reciprocates on account of pulsating air pneumatic drive signal delivered to a spring-returned piston (not shown) to which the inner tube is connected. The suction part of this instrument is also coupled to the collection container 72 by tubing 142. (Bracket 143 is intended to indicate that either tube 142 or tube 138 may be connected to the remaining portion of tube 144 which leads to the collection cassette 72.) Tubing 144 extending from the probe 140 leads to male connector plug 45 which is inserted into vitrectomy connector receptacle 146. Light 148 indicates when the connector is activated. Connector 146 supplies the pulsating air drive signal to the vitrectomy probe from a pneumatic circuit which will later be described. A conventional vitrectomy probe in the form of a guillotine cutter such as the Storz Microvit probe may be used.

Connector receptacle 150 provides access to an intraocular pressure (IOP) system, and indicator light 152 indicates when connector 150 is actuated. Connector 156 is used to deliver a pneumatic drive signal to conventional pneumatically operated microscissors (not shown), which can be operated in any one of three modes as will be further explained. Indicator light 158 is illuminated when any one of the three scissors modes is enabled. In light of the foregoing description, it will be appreciated that the three connectors 146, 150 and 156 located on rectangular plate 160 all relate to surgical functions implemented via the pneumatic system of console 46.

While certain microsurgical instruments have been illustrated or described in connection with Figure 1A, it should be understood that the microsurgical control system 40 can be used with other instruments of a similar type. In general, any microsurgical instrument that is actuated or controlled by fluid pressure (whether positive or negative), can be made to operate with the pneumatic control system of the present invention.

### d. Irrigation Pinch Valve

The irrigation pinch valve assembly 76 is utilized to provide on/off control for the gravity-infused salt solution held in the IV bottle. The pinch valve is operated by an on/off solenoid of the pneumatic system as will be further explained. Display 66, which may be an LED display or the like, indicates the height of the IV pole above the minimum reference height established via the zero switch 62e.

### 3. Off-line Memory Storage of User Data.

On occasion, it is desirable to store selected operating values or set-up parameters for a particular surgeon or microsurgical operation in off-line memory. A removable memory key 132 is provided for this purpose. The key 132 includes an integrated memory circuit which stores such operating values or set-up parameters. Console 46 receives the key 132 through a key receptacle interface 134 mounted in plate 136. Suitable types of memory keys and receptacle interfaces are commercially manufactured by Datakey, Inc. of Burnsville, Minnesota. However, it should be appreciated that other suitable means for storing particular user data may be employed with the console 46 as well, such as electronic cards with memory, magnetic disk media, or the like.

### 4. Display & Keys Of Secondary Panel (Fig. 2)

The functions associated with the secondary panel 60 will now be described. As best seen in Fig. 2, panel 60 is used to control a motorized IV pole (not shown) that supports one or more bottles or pouches of balanced salt solution used to provide irrigation during ophthalmic surgical procedures. The motorized IV pole includes a reversible electric motor/gear reducer combination which adjusts the height of the IV pole up or down as desired. The particular height may be selected via the buttons on control panel 60. Buttons 62a and 62b are used to lower and raise the pole incrementally, as long as the button is held. Button 62c is used, under emergency conditions, to send the pole upward rapidly to its maximum height, and indicator emblem 64c is illuminated when this function is activated. Button 62d, when depressed, automatically lowers the IV pole to a convenient height to facilitate changing of the IV bottle. Button 62e is called the "zero switch" because when pressed it establishes the zero reference, i.e., the minimum height for the IV pole. Button 62f and 62g are used respectively to change the height for the IV pole to either a first or second preset level. Button 62h is used during set-up to specify the first and second preset heights of the IV pole.

### B. Rear of Control Console (Fig. 1B)

Figure 1B shows the rear of the system console 46, including the rear surface 166. The console 46 includes a base frame or chassis 168, a sheet metal cover 170 having three sides forming an inverted U-shape, and back cover plate 172 occupying roughly the top two-thirds of the surface 166. The bottom one-third of the rear surface 166 is occupied by the rear wall 174 of pneumatic drawer module 44 shown in phantom, and the rear wall 176 of electrical power drawer 178 which is also partially shown in phantom and will be later described. Mounted on the upper rear cover plate 172 are the following devices: small ventilation fan 182, a large ventilation fan 184, an electrical connector receptacle 190 for a footpedal controller, an IV pole connector receptacle 192, an accessory connector receptacle 194 and a CRT screen brightness control knob 196. Cover slots 198 and 199 are also provided for future expansion to allow addition of RS232 communication ports. Rear cover plate 172 includes a set 202 of 24 ventilation louvers arranged in three columns. Rear wall 176 of power electrical drawer 178 includes a set 206 of eight ventilation louvers arranged as shown. Both sets 202 and 206 of louvers allow air to be drawn inside of the console 46. Air drawn in through louvers 202 circulates internally and eventually exits at exhaust fan 184, while air drawn in through louvers 206 is substantially confined to circulate within the electrical drawer 178 and past the lamp drawer 41 since it is confined by shelf/cover 208 and plenum 212 to be exhausted by ventilation fan 182.

The main pneumatics supply connection to pneumatics drawer 44 is made through a male Schrader quick-disconnect fitting 214 in the lower left rear corner of rear wall 174. Electrical power is provided to the electrical drawer module 178 via electrical receptacle and fuse holder assembly 218. A main on/off electrical power switch 220 for turning the console 46 on or off, is located above receptacle 218. The various hardware assemblies and drawers of console 46 are constructed in a highly modular, easy-to-assemble and easy-to-service manner.

## II. Surgical Modes & User Interface In General

### A. Switch-Selectable Surgical Modes & Features

The control console 46 is the heart and brain of the multi-function microsurgical system 40. The system 40 supports up to nine switch-selectable modes which are used in either or both anterior segment and posterior segment ophthalmic surgery. These modes are: (1) irrigation only, (2) irrigation/aspiration, (3) phaco (either emulsification or fragmentation), (4) vitrectomy, (5) controlled anterior capsulotomy (CAC), (6) bipolar, (7) scissors, (8) illumination, and (9) intraocular pressure (IOP) control. Each mode is automatically integrated into the system 40 in a manner appropriate to the type of operation selected by the operator via keys 52-58.

1. Irrigation mode employs a footpedal on/off control of irrigation. This operating mode is intended for use during an anterior capsulotomy and other anterior segment procedures in which irrigation without aspiration is desired.

2. Irrigation/aspiration mode provides foot-pedal on/off control over irrigation and linear footpedal control over aspiration. This mode is intended for use in the engagement, stripping and removing of residual lens cortical material in extracapsular cataract extraction and phacoemulsification procedures.

3. Phaco mode implements the phacoemulsification and phacofragmentation functions, which are available for both anterior and posterior segment procedures. Under phacoemulsification procedures, a "fixed phaco" mode is available in which the phaco power and aspiration levels are set via the console controls, and "linear phaco" mode is available in which phaco power is footpedal controlled and aspiration level is determined by the console controls. For phaco fragmentation procedures, a fixed phaco mode controls aspiration via the footpedal.

4. Vitrectomy mode makes the vitrectomy function available for both anterior and posterior segment procedures. For anterior vitrectomy, footpedal on/off control is provided for vitreous cutting and irrigation, while linear footpedal control is provided for aspiration. For posterior vitrectomy, this mode provides footpedal/on-off control over vitreous cutting and linear footpedal control over aspiration.

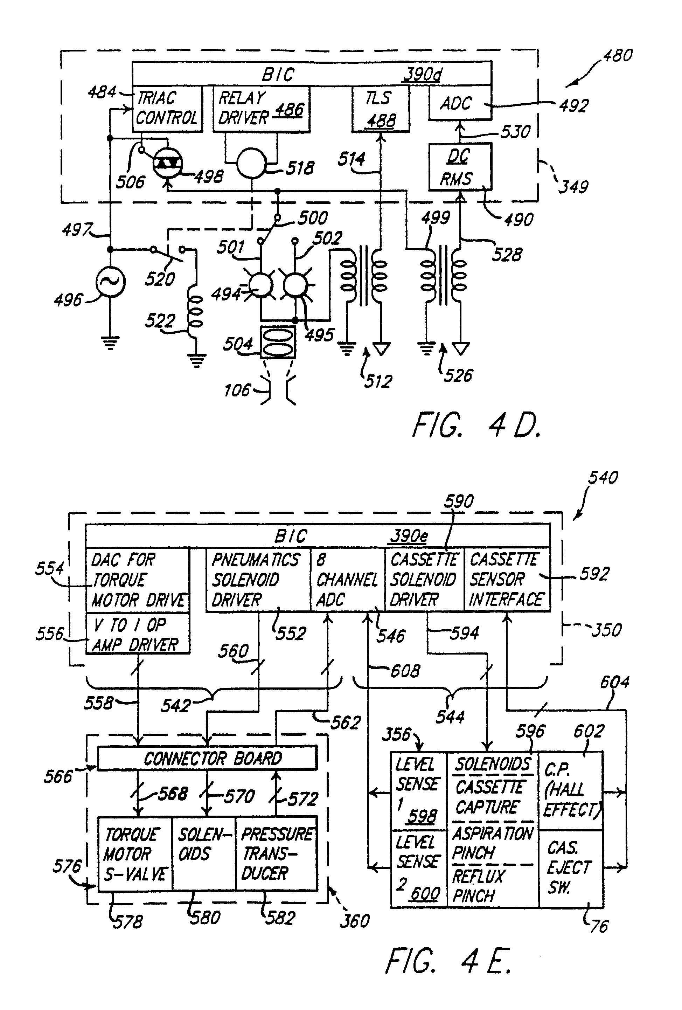

5. CAC mode provides footswitch on/off control of a CAC probe, and is explained further in connection with the discussion of Figure 4D below.

6. Bipolar mode provides on/off control of bipolar power via the footpedal assembly, and is described further in the discussion of Figure 4D below.

7. Scissors mode enables the posterior surgeon to employ a pneumatically driven intraocular scissors in any one of three foot-pedal controlled cutting operations: single cut, variable rate or proportional, which will be explained in more detail later.

8. Illumination mode provides fiber-optic illumination to facilitate viewing the posterior segment during posterior procedures. The light source thereof is adjustable from approximately five-percent illumination to full brilliance. Automatic lamp switching provides back-up illumination if the primary lamp should fail.

9. IOP mode provides precision regulated console-adjusted delivery of filtered air to the eye during posterior ocular pressure procedures. Alternatively this mode can be used to pressurize an irrigation supply to the eye for anterior procedures.

Many of the foregoing modes and features are also found in the Storz DAISY console. For example, like DAISY console, console 46 uses a disposable transparent cassette to collect aspirant during surgery. When the cassette is fully inserted into the cutout slot 70 in the console 46, the system 40 will automatically secure the cassette via a solenoid-actuated valve, and a vacuum connection will be established at that time.

10. Additional Surgical Features . The system 40 also includes additional features, namely, aspiration prime and irrigation prime in the same manner implemented in the DAISY console. Further, the special repeat reflux procedure is supported by the control console 46 in order to allow a handpiece to be cleared with pneumatic pressure if it becomes clogged with tissue. This reflux feature is available in all anterior modes, and consists of repeated reflux action.

B. User Interface Strategy

The integration of all of the aforementioned functions into a single console 46 represents a formidable organizational challenge since the system 40 must provide the operator(s) with a straightforward means of invoking all of the different modes, the functions under each mode, and a way to adjust the various set-up and operating parameters associated with various electronic control circuits and pneumatic control systems. The CRT display and pushbutton arrangement assists system flexibility greatly in this regard since it is possible to reprogram the functions of the switches 52 in accordance with the selected anterior segment or posterior segment procedure or with the selected utility functions, such as establishing set-up values or configuring the system for a particular surgeon's use.

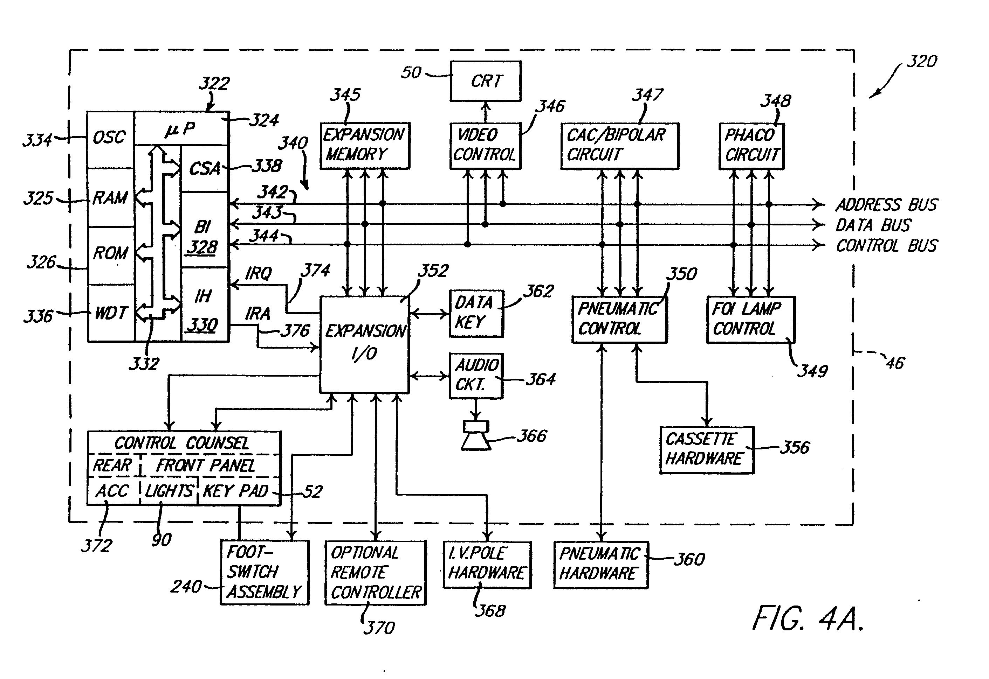

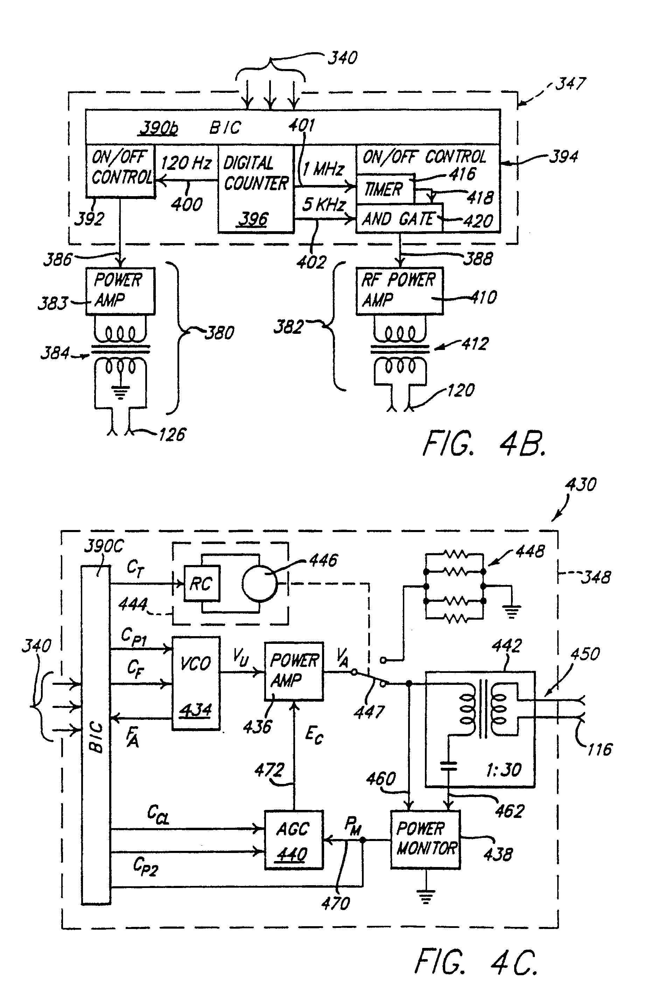

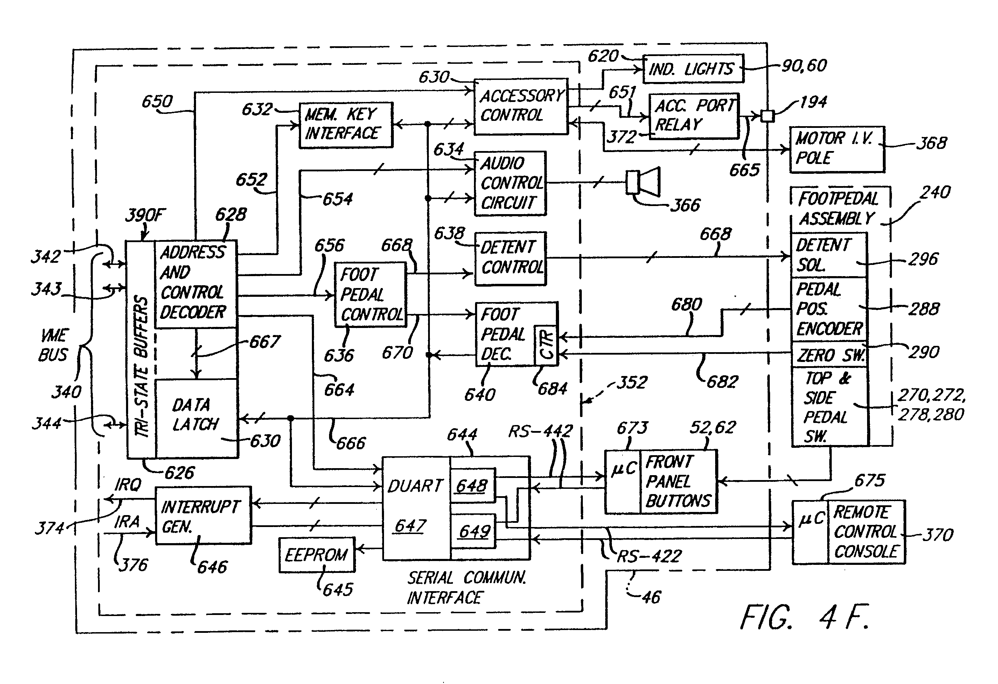

Similarly, the use of a microprocessor-based control system, described in Figure 4, enables the various strategies for the control functions to be stored in memory and called upon as required. To reduce cost of construction and assembly time, to increase reliability and serviceability, the various components of the surgical system have been constructed as separate modules or subassemblies where possible. This approach is evident in the electronics portion and pneumatics portion of the control system 40. Where practical, distinct electrical functions have been placed on their own printed circuit board which is separately addressed by the microprocessor. Similarly, the pneumatics functions have been collected and placed in one drawer module to allow easy installation and replacement.

III. Footswitch Assembly (Fig. 3)

Figure 3 shows a plan view of foot controller 240 (also called a footswitch assembly) utilized by the system 40 which has a metal carrying handle 241 and is linked directly to the console 46 with a suitable length of multi-conductor electrical cable 242 which has suitable multi-pin connector 244 at the end thereof that plugs into connector receptacle 190 on the back of the console 46. The footswitch assembly 240 includes:

a large plastic molded housing 246 enclosed with a large rectangular bottom plate 248; having a footpedal 250 which pivots about a horizontal footpedal shaft 252 supported by sintered bronze flange bushing assemblies 254 and 256; left and right vertically arranged side pedals 258 and 260; and left and right top footswitch assemblies 262 and 264 having mushroom heads 266 and 268 and electrical contact blocks 270 and 272, shown in phantom, to signal when the respective top buttons have been pushed. Side switches 278 and 280, shown in phantom, which may be microswitches or magnetic proximity switches, are actuated and provide electrical signals indicating when their respective side pedals 258 or 260 have been pressed. The housing 246 includes left and right bunker structures 282 and 284 which rise above footpedal 250 upon which top footswitches 262 and 264 are mounted. Underneath left bunker 282 is located a footpedal position encoder assembly 286 shown schematically in phantom. Assembly 286 includes an optical position encoder 288 which produces two digital signals in a quadrature relationship as the shaft 252 rotates, and a zero reset switch 290. Under bunker 284 is located a detent assembly 294 which may be electrically engaged as desired via detent control solenoid assembly 296 including an electrical solenoid coil 298. The side switches 278 and 280 and top footswitches 266 and 268 provide on-off control of certain features during selected ophthalmic procedures. For example, the left top footswitch 266 provides on/off control of bipolar coagulation. The right top footswitch 268, via display 50 and buttons 52, may be configured to control the emergency rapid-up feature of the motorized IV pole option or to control some other operating room device via the accessory receptacle 194 on the back cover 166 of console 46. In anterior segment procedures, the footpedal is used to control irrigation, aspiration, phaco and vitrectomy modes in a manner like that used for the Storz DAISY console.

IV. Electronic Control System (Fig. 4)

A. General Overview of Electronics Hardware (Fig. 4A)

Figure 4A shows a simplified block diagram of a microprocessor-based electronic control system 320 found in the control console 46 shown in Figure 1. Control system 320 includes a microcomputer 322 having a microprocessor 324, volatile (RAM) memory 325, nonvolatile (ROM) memory 326, a VME bus interface circuit or port 328, an interrupt handling circuit or port 330, and an internal control/address/data bus 332 which allows internal communications in conventional fashion between all portions of microcomputer 322. A preferred microprocessor 324 is a 68000 Series Motorola microprocessor with a clock speed of 12.5 Megahertz and one wait state for handling interrupts, although any other suitable microprocessor could be used. Computer 322 also includes a 25 Megahertz crystal oscillator 334, a watchdog timer circuit 336, and a chip select and addressing (CSA) section 338. The microcomputer 322 is located on a single board, called the processor board.

The microcomputer 322 which is located on its own printed circuit (PC) board, communicates with the remainder of the electronic control system 320 via a VME bus 340 consisting of address, data and control lines 342, 344 and 346. The VME bus 340 is used to communicate with seven other boards within the system 320, namely: the expansion memory PC board 345, the video control PC board 346 which drives the visual display 50, the CAC/bipolar circuit PC board 347, the phaco circuit PC board 348, the lamp control PC board 349, the pneumatic control PC board 350, and the expansion I/O PC board 352.

The groupings of various functions on distinct PC boards was done in order to make maintenance simpler. By clustering similar or related functions together on one board, it is possible to reduce diagnostic time and service costs since individual functions not performing correctly may be isolated on a board-by-board basis, and suspect boards may be replaced as needed. The processor board 322, the expansion memory board 345 and the video board 346 are all conventional purchased items from PEP Modular Computers GmbH of Kaufbeuren, West Germany. The manner in which all of these boards are designed and work from a hardware and operating system perspective is conventional. The manner in which the video board 346 drives the CRT 50 is conventional too. The CRT 50 used with the control console 46 is preferably a 9-inch diagonal monochrome monitor with standard resolution, although any other suitable two-dimensional display may be utilized such as liquid crystal display or electro luminescent display.

The lamp control board 349 is used to control the components in the lamp drawer 41 which is the source of light for fiber optic light pipe 100 shown in Figure 1A. Pneumatic control board 350 is used to control the cassette hardware 356 and the pneumatic drawer hardware 360. The cassette hardware 356 refers to those input devices such as switches and output devices such as solenoids associated with the aspirant collection cassette 72 shown in Figure 1A. The pneumatic hardware includes pressure transducer, a torque motor servo-valve and solenoids.

Expansion I/O board 352 is used to communicate or control the memory key circuit 362, the audio generator circuit 364, which drives speaker 366, the IV pole hardware 368, an optional remote controller 370, and the footpedal assembly 240 of Figure 2. The expansion I/O board 352 also is used to interrogate or operate various other input and output devices associated with the control console 46, such as the keypads 52, the indicator lights on secondary panel 60 and connector panel 90 and the accessory relay 372 associated with accessory receptacle 194 shown in Figure 1A. All user-generated input commands are handled through I/O board 352. To ensure such commands are promptly communicated to the processor 324, board 352 generates an interrupt request (IRQ) signal on line 374 to inform the processor 324 that the I/O board needs to be serviced. The processor also generates an interrupt acknowledge (IRA) signal on line 376. In this manner, user input commands take precedence over lower priority I/O tasks also being handled via VME bus 340.

B. CAC/Bipolar Circuit (Fig. 4B)

Figure 4B is a detailed block diagram of the CAC/bipolar circuit board and the power amplifier/transformer sections 380 and 382 which it drives with DC level control signals 386 and 388. The board 347 includes a standard VME bus interface circuit (BIC) 390b, which is interfaced directly to on/off control circuits 392 and 394 and to a multi-stage digital counter 396. The digital counter 396 is continuously run, and taps are provided at various stages thereof to provide three digital logic level, timebase signals, all of which are square waves having a 50% duty cycle, namely a 120 Hz signal on line 400, a 1 MHz signal on line 401 and a 5 KHz signal on line 402.

CAC function is best understood by explaining a few basics about the capsular anterior capsulotomy procedure. During this procedure, a pyramidally-shaped tip positioned at a right angle to and near the tip of a microsurgical needle vibrates at a fixed rate, such as 120 Hz, in two dimensions, namely axially and transversely. This cutting action is used to cut the anterior capsule of the eye. Upon receiving an appropriate command from the processor 324 over the VME bus 340, on/off control 392 allows this signal 400 to pass through to line 386. Power amplifier 383 amplifies digital signal 386 to approximately 2 watts and transformer 384 converts the output signal from the power amp 383 to a square wave which varies between plus and minus 3.5 volts. The amplitude and frequency are fixed. The power from the secondary transformer 384 is applied to connector 126 on receptacle panel 90 of the console 46. A conventional CAC probe from Storz may be plugged into connector 126.

The bipolar cautery function implemented by the board 347 is conventional, and has been used in the Storz DAISY console several years. In bipolar cautery, a high frequency moderate power signal is applied to electrodes located at the tips of a conventional bipolar probe. The high frequency electrical signal is used to cauterize severed blood vessels, incisions and the like. In the preferred embodiment, a 1 MHz power RF signal is output to connection 120 by RF power amplifier 410 and step-up transformer 412. The maximum output may be limited to 7.5 watts at 100 ohms. The power of the RF signal applied to connector 120 is preferably adjustable from zero to 100 percent. In the electronic control system 320, this is implemented in the following manner. First, the bipolar signal applied to connector 120 is considered to be at 100 percent power when the RF signal is modulated so as to be on 50 percent of the time and off 50 percent of the time. The low level 1 MHz signal 401 is pulse width modulated by on/off control 394 using the 5 KHz signal 402 as a time base. The signal 402 has a 50 percent duty cycle, which means it is on for 100 microseconds and off for 100 microseconds each cycle. This represents 100 percent bipolar power. To reduce the power level, a digital timer circuit 416 within the on/off control 394 reduces the on time of signal 402 while increasing the off time, thus resulting in a pulse width modulated (PWM) signal 418 having a duty cycle corresponding to the duty cycle required to achieve the desired power level. This signal 418 is applied to gate circuit 420 resulting in signal 388 being a PWM composite RF signal which when on oscillates at 1 MHz. Signal 388 is fed to RF power amplifier 410, whose output drives the primary of transformer 412. Transformer 412 isolates the amplifier 410 and provides proper output impedance levels at its secondary. On/off control block 394 thus regulates when the composite RF signal 388 is on, and its effective duty cycle.

## C. Phaco Calibration & Drive Circuit (Fig. 4C)

Figure 4C shows a detailed block diagram for the phaco circuit 430 forming part of electronic control system 320. The phaco circuit includes another standard VME BIC 390C, which is used to produce a variety of digital control signals including the test command signal $C_T$, a first power command signal $C_{P1}$, a closed loop command signal $C_{CL}$ and a second power command signal $C_{P2}$. The phaco control module includes a voltage controlled oscillator (VCO) section 434, a power amplifier section 436, a power monitor section 438, an automatic gain control (AGC) section 440, a transformer section 442, a relay control section 444 including relay coil 446 which operates a Form-C electrical contact 447 and a resistor bank 448. The phaco drive circuit 430 produces an ultrasonic (US) signal 450 which ranges in strength between 0 and 35 watts at a frequency in the range of 26 KHz to 31 KHz at approximately 5 kilo-ohms. This ultrasonic signal is applied to connector 116 of the receptacle panel 90 of console 46. A conventional phacoemulsification or phaco fragmentation probe may be provided power by plugging its electrical jack 114 into receptacle 116.

In operation, the phaco drive circuits 430 checks itself by having relay section 444 switch the contact 447 to its opposite position, thus applying the US signal from power amplifier 436 to resistor bank 448. Next, circuit 430 switches relay coil 446 off, thus allowing power to flow from amplifier 436 through contact 447 to transformer section 442. At this time the dominant resonant frequency of the ultrasonic transducer is determined by monitoring the voltage and currents signals on conductors 460 and 462 as a US test signal $V_A$ is swept through frequencies within the range of 26 KHz to 32 KHz. During this time, processor 324 looks for power peaks, among other things, to find the resonant frequency. Once the dominant resonant frequency of the transducer/probe plugged into connector 116 is determined, the phaco drive circuit 430 enters a drive mode. In this mode, the circuit 430, under user commands interpreted by processor 324 and delivered via VME bus 340, drives the VCO section 434 at the dominant resonant frequency and desired power level indicated by commands CF and $C_{P1}$ which is passed along as a voltage signal $V_U$ to power amplifier 436, where it is amplified and transferred as signal $V_A$ to transformer section 442. Power monitor section 438 observes the voltage and current applied to the primary of transformer section 442, and produces the monitored power signal $P_M$ on line 470, which feeds into AGC section 440 where it is compared against the desired power command $C_{P2}$. Any deviation between the power desired and the monitored power results in a non-zero error correction signal $E_C$ on line 472, which alters the gain of power amplifier 436 to compensate for and eliminate this error. In this manner, constant power operation of the ultrasonic transduce/probe combination plugged into receptacle 116 is assured.

D. FOI Lamp Control circuit (Fig. 4D)

Figure 4D shows a block diagram of the fiberoptic illumination (FOI) lamp control circuit, which includes the lamp control board 448 shown in Figure 4A, and the electrical hardware 480 controlled by PC board 349. The board 349 includes a triac controller 484, a relay driver circuit 486, a threshold level sensing (TLS) circuit 488 an RMS-to-DC converter 490 and 8-bit resolution analog-to-digital converter (ADC) 492. Conventional opto-isolators are used in circuits 484 and 486 to help prevent electrical noise for these two circuits from being passed to other parts of the electronic control system 320.

The lamp drawer 41 (see Figure 1A) includes two lamps 494 and 495 shown in Figure 4D which are powered by a low voltage (15 volts RMS) AC signal source 496 which has its power delivered via conductor 497 to triac 498, conductor 499, Form-C relay contact 500 and then to conductor 501 or 502. During normal operation, the primary bulb 494 is employed to illuminate through a conventional focusing lens 504 receptacle 106 of front panel 90. Processor 324 provides signals via VME bus 340 to the lamp control board 449 instructing triac controller 484 as to how brightly to turn on the light bulbs 494 or 495. This is accomplished in conventional manner by the timing of the gate signal on line 506 applied to triac 498.

Isolation transformer 512 is used to monitor the light bulb current to determine if the lamp circuit is operating properly. Current passing through either bulb 494 or 495 also passes through the primary of transformer 512, causing a voltage to be developed across its secondary which is delivered by conductor 514 to TLS circuit 488, which produces an output when the sense current exceeds a predetermined threshold level. Processor 324 periodically checks to see if the output of TLS circuit 488 is on, which indicates that the bulb circuit is operating satisfactorily. If this signal should be absent, relay driver 486 energizes relay coil 518 which transfers the Form-C contact 500 so that power from triac 498 is delivered via conductor 502 to the second light bulb 495. In this manner, lamp driver circuit 480 automatically switches to the secondary lamp source 495 when the primary bulb 494 fails to operate for any reason. At the same time, electrical contact 520 closes and rotation solenoid 522 causes a mirror (not shown) to rotate into position so that light from secondary bulb 495 shines directly into focusing lens 504.

Isolation transformer 526 monitors the voltage on line 499, which is equal to the voltage applied across the light bulb. This voltage signal induces a corresponding current in conductor 528 connected to RMS/DC converter 490 which produces a DC signal on line 530 proportional to the amplitude of the signal on line 528. ADC 492 converts this into a digital value which is transferred via BIC 390d and VME bus 340 to processor 324. Processor 324 periodically examines this value to determine whether fluctuations in the applied voltage level of the bulb are occurring. If they are, processor 324 issues appropriate compensating commands to triac controller 484, thus keeping the effective power applied to the light bulbs constant, to help ensure a constant level of illumination in accordance with the illumination level setting selected by a user via keys 52.

E. Electrical Circuit For Pneumatics System (Fig. 4E)

1. Pneumatics Control Circuit

Figure 4E is a block diagram of the electrical control circuitry 540 found on pneumatic control board 350, which as shown in Figure 4A is used to drive the electrical devices forming part of the cassette hardware 356 and the pneumatic hardware 360. The circuitry 540 includes a pneumatic control section 542 and a cassette control section 544. The BIC 390e and an 8-channel ADC 546 are common to both sections. Pneumatic control section 542 include a solenoid driver circuit 552 and a DAC 554 and a voltage-to-current op amp driver circuit 556. Three sets of conductors 558, 560 and 562 deliver signals from section 542 to a common connector board 566 located at the pneumatics drawer 44. Connector board 566 serves as a convenient termination point for three sets 568, 570 and 572 of internal conductors which run between connector board 566 and the actual electrical devices 576 being driven or read. The devices 576 include a torque motor servo valve 578 and set 580 of solenoids which operate valves and a set 582 of pressure transducers. The torque motor servo valve 578 is used to provide a proportionally metered flow of pressurized air which is used to create a desired level of vacuum for aspiration or of air pressure for operating microscissors. The rate of air flow is proportional to the opening in the valve, which is proportional to the electric current supplied to the torque motor valve 578. Processor controls this current level by sending appropriate control signals over VME bus 340 to the BIC 390e in board 540 which causes DAC 545 to generate a specified voltage level. This voltage level is converted by op amp driver 556 into an amplified current signal passed along conductors 558 and 568 to servo valve 578. Processor 324 also controls the operation of solenoid valves in the pneumatic system 44 by sending appropriate signals to BIC 390e shown in Figure 4E, which turns on individual driver circuits, as desired in solenoid driver's circuitry 552. Thus suitable voltage signals (such as 12 volts DC) are applied along individual ones of conductors 560 and 570 to turn on desired ones of the solenoids 580.

Pressure transducers 582 generate low voltage analog signals which are routed up through conductors 572 and 562 to respective individual channels of ADC 546, which read the analog signal levels. Processor 324 polls ADC 546 periodically through BIC 390e to obtain digital values of the pressures sensed by transducers 582.

## 2. Cassette Control Circuit

Cassette control section 544 includes conventional solenoid driver circuitry 590 and sensor interface circuitry 592. Solenoid drive circuit 590 provides amplified voltage signals to three solenoids used to operate two-position, three-way pneumatic valves that individually control three small pneumatic cylinders used for cassette capture, aspiration pinch and reflex pinch operations. The cassette hardware 356 includes two level sensing devices 598 and 600 which detect when fluid in the collection cassette 72 has reached predetermined levels one and two corresponding to "cassette nearly full" and "cassette full" fluid levels. Hardware 356 also includes a Hall effect switch 602 (used to detect the presence of the spring-loaded mechanical lever which is pressed when the collection cassette 72 is fully inserted in slot 70) and the cassette eject switch 76 shown on panel 190 in Figure 1A. Sensor interface 592 reads the electrical signals on conductor 604 to determine the states of devices 602 and 76. Two channels of ADC 546 read the states of level sensing devices 598 and 600 over conductors 608. Periodically, processor 324 interrogates sensor interface 592 and ADC 546 to determine the status of sensing devices 598, 600, 602 and 76. The level sensing device 598 preferably consists of a LED and phototransistor positioned on opposite sides of the cassette 72. As the liquid level rises, a plastic ball which floats rises as well and breaks the light beam between the LED and phototransistor. The level sensing device 600 preferably consists of the same type of LED/phototransistor arrangement, but located at a slightly higher level.

## F. Expansion I/O Board Circuit (Fig. 4F)

### 1. Introduction

Figure 4F shows a detailed block diagram of the I/O expansion board 352 and the devices which it drives or reads, namely: the indicator lights on secondary front panel 60 and on connector panel 90 (represented by block 620) and relay 372 for accessory connector 194, the speaker 366, and the primary and secondary front panel buttons 52 and 62, ala of which are located in or on the control console 46 (indicated by these devices being to the left of the dashed line 46, which represents the perimeter of control console 46). The board 352 also drives and/or reads devices in the motorized IV pole assembly 368, the footpedal assembly 240, and the optional remote control console 370, which are all outside of the control enclosure 46.

The I/O board 352 communicates with the VME bus 340 through a VME interface 390f which includes tri-state buffer circuits 626, address and control decoder circuit 628 and 16-bit data latch or register 630. The I/O circuitry on board 352 also includes four primary control interface circuits, namely accessory control 630, memory key control 632 for memory key 132, audio control 634 for speaker 366, and footpedal control 636. Control circuit 636 in turn directs the operation of two slave circuits, namely detent control 638 and footpedal decoder 640 which actually communicate with devices in footpedal assembly 240. Board 352 also includes a conventional serial communications interface circuit 644 which drives and reads in conventional fashion an interrupt generator circuit 646 and a non-volatile memory 645, which preferably is an electrically erasable programmable read only memory (EEPROM). Circuit 644 includes three conventional integrated circuit (IC) chips, namely a dual universal asynchronous receiver/transmitter (DUART) 647, a dual-channel RS-422 transmitter chip 648, and a dual-channel RS-422 receiver chip 649, all functionally connected as shown in Figure 4F.

The primary interface circuits 630-638 and the serial communications interface 644 communicate with VME bus interface 390f via control signals passed along dedicated control lines 650-656 and 664. Data to be sent to and/or received from circuits 630-638 or communications interface 644 is passed along an internal 16-line data bus 666 connected to data latch 630. Footpedal control 636 communicates with slave circuits 638 and 640 via lines 668 and 670. Each of the primary control circuits and the communications interface 644 contains a data latch circuit for receiving, holding and/or transmitting data to internal data bus 666.

Address and control decoder 628, upon receipt of commands from processor 324 via VME bus 340, decodes the command and address signals on lines 342 and 343, and in accordance with the decoded instructions distributes the desired control signals and/or via lines 667 commands data signals to the control interface circuit 630-636 or 644 which the processor 324 desires to address. The control interface circuits 630-636 have no intelligence and do not on their own seek to communicate with processor 324. Instead, processor 324 just periodically writes or reads data to these control circuits.

## 2. Functions of Serial Communications Interface 644

The communications interface 644 has two devices connected to it which have intelligence, namely microcontroller 673 associated with the two front panels 48 and 60 on console 46 and microcontroller 675 associated with optional remote controller 370. Serial communications interface 644 converses with the microcontrollers 673 and 675 using the well-known RS-422 communications protocol at a suitable data rate, such as 9600 baud. Whenever either of these two microcontrollers has information to be sent to processor 324, it serially sends a byte of information to the communications interface 644 which in turn automatically causes an interrupt to be generated. Communications interface 644 is identified as the source of the interrupt, the interrupt is acknowledged via line 376, and the processor 324 causes data serially communicated to the DUART 647 by the microcontroller to be loaded into the data latch 630, and then via VME bus 340 reads the data from latch 630 in one of its next I/O cycles. Microcontroller 673 has its own internal oscillator and micro program. It continuously monitors all of the buttons 52 and 62 found on front panels 48 and 60 of the control console 46 to determine whether they have been depressed. The buttons are electrically arranged in a matrix of row and columns, and by interrogating each position of the matrix the state of all the buttons is determined. The microcontroller advises the processor whenever a button is pressed, and keeps periodically advising the processor 324 of this fact for as long as the button remains pressed. Microcontroller 673 also monitors, as part of the aforementioned matrix of buttons, the status top buttons 270 and 72 and side pedals switches 278 and 280 within the footpedal assembly.

The microcontrollers 673 and 675 are provided in order to ensure that the main processor 324 is apprised of changes in status at the front panel console or remote control console virtually immediately for a very quick response to operator requests. In other words, all the routine functions which need not be performed quickly by the main processor 324 are made to wait while processor 324 responds to an interrupt and reads the data from the microcontroller and puts it into a table in main memory 325 one byte at a time. In main memory, a table listing the states of all the buttons on the main console and the remote control console is kept. The microcontrollers 673 and 675 only advise the main processor 324 of changes in the state of any of the buttons. In this manner, communications between the microcontrollers 673 and 675 are handled far more efficiently than updating the entire table each time an interrupt is generated.

Microcontroller 675 operates in the same manner as microcontroller with respect to the matrix of buttons it monitors. The remote control console 370 also contains a keyboard interface circuit almost identical to interface circuit 680.

Communications interface 644 also reads and writes data to EEPROM 645 in conventional fashion. EEPROM 645 is provided so console 46 can store, in a non-volatile manner, any user-programmed default values, configuration codes, calibration data and/or any other pertinent parameters which may be entered in by the user.

## 3. Accessory Control Circuit 630

The accessory control 630 contains: a plurality of memory latches and indicator light driver circuits dedicated to driving the indicator lights 620 on connector panel 90 and secondary panel 60; a plurality of memory latches, relay driver circuits, sensing circuits and an optical position decoder, all of which are dedicated to sending control signals to and receiving information from motorized IV pole hardware 368; and a latch and relay driver for operating relay 372. When a relay driving signal is applied to line 662, relay coil 372 is energized, which closes a normally open contact and thus completes the circuit available on lines 664 connected to the connector receptacle 194 shown in Figure 1B.

## 4. Audio Control circuit 634

The audio control circuit 634 is of standard design, and uses a conventional programmable sound generation circuit on a large scale integration (LSI) chip to produce the various tones at various amplitudes used to indicate device operation and provide audio error signals to the console user. The output signal from this chip drives a separate conventional low-power audio amplifier chip, whose output is connected to and drives speaker 366. A suitable sound generator is available from Microchip Technology, Inc. of Chandler, Arizona as Model No. AY8930. One of the unique features provided by control console 46 is the user of select various tones and amplitudes for the selected tones to represent different conditions or states that the control system 40 may be placed in by the surgeon.

## 5. Footpedal Control Circuits 636-640

In accordance with commands from footpedal control 636, the detent control 638 provides positive and negative 24 volt DC power signals on lines 668 to operate the detent solenoid 698. A momentary +24 VDC signal extends the armature of solenoid 688 while a momentary -24 VDC signal causes it to retract. Conventional magnetic and/or mechanical detents built into solenoid 698 hold its armature in the last position the signals on lines 668 placed it in.

Footpedal decoder 640 receives low-voltage quadrature signals over conductors 680 from encoder 288, and a low-voltage digital signal on line 682 from zero switch 290. Switch 290 is released whenever footpedal 250 is moved more than two degrees from its spring-returned position, that is, the position pedal 250 is in when it is not pressed at all. When the signal on line 682 is in its reset state, bi-directional multiple stage digital counter 684 with-in decoder circuit 640 is held in a reset state. As soon as signal 682 goes to its opposite state, counter 684 is allowed to operate under the control of the quadrature signals on lines 680 which increment or decrement the counter with each pulse. Thus the accumulated count in counter 684 reflects the true angular position of footpedal 250. Processor 324 periodically (once every 50 milliseconds) reads the value in counter 684 by sending appropriate control signals to bus interface 390f so that counter 684 can send its present count to data bus 666, where it is held by latch 630 until read by the processor 324 via VME bus 340.

The status of top button switches 270 and 272 and the side pedal proximity switches 278 and 280 of the footpedal assembly 240 are also read through microcontroller 673, which as previously explained serially transmits information to communications interface 644, through internal bus 666, bus interface 390f and VME bus 340 to processor 324.

## V. The Improved Operator Interfaces Of Control Console 46 (Figs. 15-18)

In the control system 40 of the present invention, the display 50 on control console 46 is used to present to the user a number of different menus in a predetermined arrangement. This procedure is also employed in the Storz DAISY console, but in a much more limited fashion. Through the diagram presented in Figure 5, the significant differences between the enhanced operator interface of the present invention and that employed in the DAISY console should become apparent to one of ordinary skill in the art.

In Figure 5, each separate menu or display screen forming part of the tree structure 700 is shown in a separate Figure, and the Figure number is indicated in parentheses within the block. The first screen which the user normally sees in the system 40 of the present invention is the main or sign-on screen represented by block 710. From this screen the user may as indicated by branch path 712 may go to the main anterior segment screen 714, the main posterior segment screen 716 or the "utilities" screen indicated by block 718.

Once in the main anterior segment screen 714, the user as indicated by branch 722, may select the screen for the irrigation/aspiration mode indicated by block 724, the screen for fixed phaco mode indicated by block 726, or the screen for linear phaco mode indicated by block 728.

If the user selects the main posterior segment screen 716, then branch 732 indicates that the user may choose from screen 716 to enter the illumination mode screen indicated by block 7.34, the screen for the IOP control mode 736 (which will be explained via Figure 8B), or the menu screen for vitrectomy mode indicated by block 738.

If the utilities mode screen 718 of the present invention is selected by the user then the multiple-path branch 740 indicates the user may go to any one of nine different setup screens including the following: interior input settings screen 742; posterior input settings screen 744; aspiration setup screen 746; vitrectomy setup screen 748; language setup screen 750; audio control setup screen 752; accessory setup screen 762; custom title setup screen 764; or power-up mode setup screen 766. From the power-up mode screen 766, a user may select to go, as indicated by branching lines 770, to power-up mode setup screen 772 or power-up posterior tool setup screen 774.

Figure 6 shows the main sign-on screen 710 and the collection 52 of membrane switches or buttons arranged in left and right columns 54 and 56 and bottom group 58, all located on the primary front panel 48 of control console 46. Figure 6 also shows in dotted lines the fields of left side display region 84, right side display region 86, bottom display region 88 and top display region 92. Region 92 is comprised of an upper field 94 and lower field 96, sometimes referred to as the primary and secondary headings. In addition, the central field region 82 is also shown. As will be made more clear hereinafter, the various fields in these different display regions can and do hold different legends. In the Figures of the present application, these legends are set forth in the English-language, but those skilled in the art will appreciate that they may be provided in virtually any other language as well, or possibly in universal symbols recognized independently of the language spoken by the user. This is one of the significant advantages of using a display 50 such as a CRT to which all sorts of different information may be presented.

The side regions 84 and 86 each consist of six horizontal fields stacked one above the other and positioned to correspond to the locations of the buttons of button groups 54 and 56. By virtue of the adjacent location of the top button 54a of the button group 54 and the top field 84a of the region 84, for example, a message or legend in the field 84a is readily understood by the operator user as referring to the uppermost button 54a. The other buttons in fields are similarly paired. This arrangement allows indicated function of each of the buttons 54 or 56 to be readily changed by simply changing the legend displayed in its adjacent field. In a similar manner, each pair of buttons (such as buttons 58a-1 and 58a-2) is associated with one of the three-part fields along the bottom region 88 (such as region 88a). In general, the upper row of the buttons 58a-1 through 58e-1 are used to increment a setting or parameter of the type displayed in the corresponding fields 88a-88e of the display 50 directly above, while the buttons in a lower row, i.e., buttons 58a-2 through 58e-2, are used to decrement the displayed settings or parameters in the corresponding three-part 88a through 88e directly above. The button 58f labeled "INFO?" is used to bring up an information screen on the display 50 to assist the operator, such as by further explaining functions associated with the current choices on the display menu. Button 58g is to return to an earlier menu screen in a chain of related menus or other screens, such as the tree structure 700 shown in Figure 5. When button 58g is pushed while in one of the utilities set-up menus, processor 324 stores any parameters or other settings that may have been altered while in the set-up menu.

In Figure 6, the main screen 710 shown in display includes a copyright notice in the lower field 96 of upper display region 92, the assignee's name in the central region 82, and three functional legends located at fields 84a, 86a and 86f respectively labeled "ANTERIOR," "POSTERIOR" and "UTILITIES." The selection of one of the corresponding buttons such as 54a, 56a or 56f causes the processor 324 to present on display 50 the next screen in the chain of screens as indicated by the depressed button. Thus, for example if the button 54a is depressed, processor 324 clears screen 50 and presents the screen shown in Figure 7A.

### A. Anterior Menus (Figs. 7A-7D)

Figures 7A-7D show a representative group of the anterior segment menus used during various anterior segments surgical procedures. Figure 7A bears the legend "ANTERIOR SEGMENT" in field 92. The bold lettering is used to indicate that the surgical modes or functions described by these legends, which are in fields 84a-84f, 86a and 88b, may be selected. Specifically, any one of these functions, such as irrigation/aspiration mode whose legend is shown in field 84b, may be selected, for example by the user depressing button 54b. The legend in fields 88c are shown in faint lettering because these surgical procedures may be performed from one of the anterior segment menus, but not from the main anterior menu display screen 714 shown in Figure 7A.

Of the functions shown in the legends in bold lettering on display screen 714, all were provided in a similar fashion on the CRT display of the DAISY console, except for the IOP control mode indicated by legend 84f and the phaco calibration procedure indicated by legend 88f. Thus, the old modes familiar to those in the art through the DAISY console will not be described more than they already have, except that display menus 724-728 in Figures 7B-7D and 8A-8C will be discussed in order to acquaint the readers unfamiliar with the DAISY console menus of how the menu structure of the DAISY console generally appears, and where various parameter legends and how other informative legends typically appeared.

Figure 7B shows the irrigation/aspiration screen 724 in field 50. All of the active buttons and mode selection which are capable from this menu are listed in bold lettering in the fields adjacent the buttons which would activate such modes.

The exit switch 58g is functional only when a mode has not been selected. If the exit switch is pressed, the display will return to the prior screen. The info switch 58f is functional in all modes at all times. The info switch may be pressed at any time to obtain information concerning any mode or operation without interrupting the operation in progress.

The primary modes are displayed in the left regions 84 of the anterior menu 724. Primary modes are: irrigation; irrigation; aspiration/phaco, and vitrectomy. Only one primary mode may be selected at a time. The secondary modes are: bipolar, IOP control, and CAC. The secondary modes can be selected for use with a primary mode, or by themselves. Whenever any selection is made, that mode or function will be displayed with inverse video (as is indicated by the shading provided in field 84b when key pad 54b was depressed). The two independent features, irrigation prime and aspiration prime, allows set up by the nursing staff. They may be selected at any time unless the footpedal 250 of footpedal assembly 240 (see Figure 3) is pressed.

The aspiration function of the present invention has a programmable rise time, as will later be described with respect to Figure 12. However, it is worth noting that the central region 82 contains two display subregions 780 and 782 having display fields 780a and 780b, and 782a and 782b. The field 780b contains the legend "Asp" when the aspiration function is enabled, and the field 780a which reads "Rise Time:1" indicates the rate of change of the aspiration which has been programmed into the present invention. As will later be explained, the preferred embodiment of the present invention provides six choices for rise times, although a greater or lesser number may be provided.

Field 782a provides the reading of aspiration vacuum level from a transducer which is being monitored by processor 324, and field 782b provides a legend indicating what the number presented in field 782a relates to. Further, display region 88e has its three fields provided with information therein regarding the activated ability of buttons 58e-1 and 58e-2 to be used to raise or lower the maximum aspiration level allowed, which is presently shown as being set at 30 mmHg by the fields 88e-1 through 88e-3. Thus, the user may tell at a glance from display region 780 and 782 the status of the aspiration function in control system 40.

Figure 7C shows the fixed phaco display screen 726, which can be noted from the phaco legend and field 54c being displayed in reverse video. Further, central region 82 of the screen 50 includes a screen legend field 786, two display regions 788 and 790, arranged in columns, and a horizontal display region 792 having four fields 792a-792d. Fields 788b and 790b respectively indicate the actual parameter which is displayed in fields 788a and 790a. The fields within region 792 indicate which of the functions which are activated, by showing the functions in reverse video. Thus from region 792 shown in Figure 7C, the user knows that the fixed phaco mode is active and the irrigation, aspiration and phaco power functions are all operating. The legends and values in display regions 88a, 88c-88e provide further information about the settings relating to the functions which may be invoked in the fixed phaco mode, which have been earlier explained.

Figure 7D shows the linear phaco mode menu 728. The linear phaco mode is entered by depressing the phaco switch 54c again when menu 724 of Figure 7B is displayed. The linear phaco mode has several different parameter settings, the meanings of which should be clear from earlier discussion herein, from operation of the DAISY console, and from the reader's general knowledge of the ophthalmic surgical art.

Figure 8A shows the main posterior segment menu 716 and the modes or functions which can be selected therefrom. Once again the bold lettering in the fields of regions 84 and 86 indicates functions which can be activated immediately, while the lighter dotted lettering of legends in fields 86d and 86f indicates functions which are used in posterior segment operations but not available from this screen. When illumination mode is selected via button 54e, the menu screen 834 shown in Figure 8B results. In other words, the legend field 84e is shown in video-reverse, and the "intensity 100%" information shown in display region 88b is shown.

When the IOP control button 54f shown in Figure 8A is selected, then the alternate screen 736, also represented in Figure 8B is presented. In the IOP control screen, the IOP control field 84f is shown in reverse video, the legend field 886 is illuminated to show the maximum IOP pressure which the processor 324 will be allowed to create. upper sub-region 794 displays "IOP" in field 794a. The actual reading of IOP pressure is accomplished by a transducer being monitored by the processor 324 which reads same, and converts the reading into the units shown in the field 794b.

The legend in field 86e is shown in bold, meaning that from display screen 734 or 736, the "aspiration prime" function can be selected. When the aspiration prime function is selected, additional legends in display subregions 780 and 782 are illuminated as previously explained with respect to Figure 5 there. Thus, there is a similarity or pattern between the various menus which the user can become familiar with.

Figure 8C, which shows menu screen 738 associated with the vitrectomy mode further bears this out. In this mode, the regions 780 and 782 are once again illuminated since aspiration is required with a vitrectomy probe. Further display region 92 says "Cutter On" (or will say "Cutter Off" if the vitrectomy feature is disabled). Note that the display regions 88d and 88e advise the user of console 46 of the pre-established cut rates for the cutter and the maximum aspiration setting, both of which can be adjusted via button pair 58e.

B. Utilities Functions (Figs. 9-18)

Figure 9 shows the main utilities menu 718. From the main screen 710, the utilities screen 718 is reached by pressing button 56f. The bold lettering in legend fields 84a-84e and 86a-86d indicates that nine different specific utility menus may be reached through the main utility menu. For example, pressing button 54a takes the user to the menu 742 described in Figure 10.

The utilities options allow the user to customize the microsurgical system 40 for a particular medical procedure, practice or language. The different utilities should preferably be completed at installation; however, the utilities may be changed at a later date if desired by the user. Just one parameter or several may be changed at the discretion of the user. Throughout the utilities set-up procedures described with respect to Figures 10-18C, any entry on a screen appearing in faint lettering indioates an item or category which is not accessible from the screen which is displayed or indicates an item which has no selectable features.

From the main screen 710, the utilities screen is reached by pressing button 56f.

Figure 10 shows the anterior input settings screen 742, which is reached by pressing button 54a from the Figure 9 menu. When in screen 742, selecting button 54b allows the maximum value of aspiration to be preset for the irrigation/aspiration mode. The aspiration control will be identified above one of the input switch pairs 58a, such as in field 88e above button pair 58e, as was shown in Figure 7B. If the up arrow 58e-1 is pressed, the displayed value will increase. If the down arrow 58e-2 is pressed, the displayed value in field 88e-2 will decrease.

Button 54c, when pressed causes processor 324 to display initial values found for the fixed phaco mode, which can then be preset. The location of the three parameters, namely power level, pulse rate and fixed aspiration are in the same place as Figure 7C. When button 54c is hit a second time, the initial settings for the linear phaco mode are displayed in display regions 88b-88e as shown in Figure 7D.

When button 54d is depressed, the initial values for the vitrectomy mode may be preset. Cutting rate and maximum aspiration level are displayed in columns 88d and 88e above button pairs 54d and 54e as in Figure 8C.

When button 54e is depressed, the screen allows the bipolar level to be preset for the bipolar mode by displaying the legends shown in Figure 10 in region 88b so that buttons 58b may be allowed to adjust same.

When button 54f is depressed, the IOP control pressure level may be preset for the IOP control mode. To facilitate this, the display 742 provides in region 88a the legends shown in Figure 8B.

Figure 11 shows the posterior input settings screen 744 which functions in a manner exactly like that just described with respect to screen 742 in Figure 10. The screen 744 is reached by pressing the button 54b when the Figure 9 screen is displayed.

Figure 12 shows the aspiration setup screen 746, which is reached by pressing the button 54c when the Figure 9 is displayed. In screen 746, six possible rise times for aspiration are shown in fields 84a through 84f. The desired rise time value is simply selected by pushing the corresponding button in column 54. If the "one second rise time," as indicated by the legend in field 84a, is selected, the aspiration level will increase (or decrease) from minimum to maximum (or from maximum to minimum, if decreasing) as rapidly as possible. If the "6 SEC RISE TIME" legend is selected, the aspiration level will change at a much slower rate. The rise time values may be programmed into the processor as desired for each one of the fields 84a-84f. In other words it is not necessary to limit the possible choices to the values shown. The rise time refers to the time it takes for the aspiration control circuit to respond to a full-scale step input.

As earlier explained, the control console 46, like the DAISY console, employs a conventional closed loop servo system for maintaining the actual- aspiration (vacuum) level at the desired aspiration level selected by the user. The modification of the rise time does not alter the closed cloop control system used to ensure that the actual vacuum level produced equals the desired vacuum level requested by the user. Instead, the software within processor 324 simply conditions, if necessary, the rate of change of the desired aspiration level command signal which is input into the control loop just described. In other words, if the user steps on the footpedal 250 quickly, thus producing a step-like increase in the desired aspiration level, the processor 324 simply ramps up the actual signal provided to the control loop at no more than the rate dictated by the rise time selected via aspiration set-up screen 746 in Figure 12. In this manner, the control loop is free to operate quickly to reduce any error signal or disturbance created by factors other than quick changes in the user's command signal indicating the desired aspiration level.

One benefit of the console 46 having an adjustable rise time for the aspiration level is that the number of surgeons, particularly those who have used peristaltic pumps, do not feel entirely comfortable with the quicker rise times typically found in aspiration systems powered by an air-to-vacuum converter. By allowing such surgeons to slow down the rise time to moderate levels such as 3 or 4 seconds or even slower levels such as the 6 second rise time, the control system 40 of the present invention can closely emulate the aspiration performance characteristic of peristaltic pumps. Thus, this one user-selectable adjustment allows ophthalmic surgeons use to different types of equipment to customize control system 40 to their liking. Note that buttons 56 and 58a-58e are not used in conjunction with the display screen 746.

Figure 13 shows the vitrectomy setup screen 748, which is entered by pressing button 54d when the Figure 9 screen is displayed. The vitrectomy setup screen 748 allows selection of two vitrectomy modes. The "STORZ VIT" mode whose legend is indicated in field 84a is characterized by the right side footpedal 260 enabling and disabling the cutting action. The "MICROVIT VIT" mode whose legend is shown in display field 84b is characterized by a different pattern of operation of the footswitch assembly 240 shown in Figure 3, namely the right side footswitch 258 enables cutting, and the left side footswitch 260 disables cutting. The default selection is "STORZ VIT" so the user must utilize menu 748 if the "MICROVIT" footswitch operating characterics are desired.

Figure 14 shows the language setup screen 750, which is entered by pressing button 56c when in the Figure 9 screen. When in this setup screen 750, the user has a choice of 5 different languages indicated in display fields 84a-84e. The desired language legends to be displayed via the various display regions and fields in display 50 will then all be in the language selected. All of the legends in different languages are stored in the expansion memory 345 (shown in Figure 4A) and are readily accessed as necessary by microcomputer 322. If desired, the pertinent legends relating to the currently displayed screen may be saved in RAM 325 of the microcomputer 322.

Figure 15 shows the audio control setup menu 752, which is entered by pushing button 56d when the Figure 9 menu is displayed. The left side fields 84a-84d list the different types of tones which are available, and which may be invoked by pressing corresponding buttons 54a-54d as explained below. The legends 86b-86f indicate that five different tones are available by depressing corresponding buttons 56b-56f. Button 56a turns off the tone as noted by legend 86a. Each one of the tones 1-5 is at a different pitch, and if desired, microcomputer 322 can be programmed in conventional fashion to make each of the tone sounds distinctive, such as a clear tone, a fuzzy tone, a high pitch tone, or any combination of tones or other sounds. The audio control circuit shown in block 634 of Figure 4F is of conventional design as previously noted and a wide variety of different sounds can be produced by following instructions provided with the commercially available integrated circuit sound generating chip previously referred to. Generation of tones or other sounds or noises via a computer system is well known, and thus by itself does not form part of this invention. Rather this invention applies this known sound-generating technology in a unique manner which allows a user to customize the tones or sounds used in ophthalmic surgical procedures for monitoring purposes or warning purposes.

To select a tone for aspiration, the button 54a is depressed, resulting in legend 84a being displayed in reverse-video. Next, one of the aspiration tones is selected via buttons 56a-56f. As each tone is selected, the tone is generated and output via speaker 366 for the user to hear. The input switch pair 58c below the volume adjustment display in region 88c allows the user to adjust the loudness of the tone up or down as desired.

In a similar manner, the tones assigned for either phaco or frag mode can be selected by depressing button 54b and following the procedure just outlined. The bipolar power tone is similarly selected via button 54c.

The warning tones may come on for any condition which a designer of the processor 324 wishes to utilize. The specific warning tone for such selected conditions is selected via button 54d and following the procedure just outlined above.

Figure 16 shows the accessory setup screen 762, which is entered by pressing button 54e when the Figure 9 menu is displayed. Screen 762 provides the user with 2 choices for how the right top pushbutton 264 on the foot assembly 240 (see Figure 3) is used. This menu 762 allows the status of pushbutton 264 to control if desired the accessory port relay 372 connected to accessories connector port 194 (see Figure 1B and 4F) by depressing button 54a. If button 54b is depressed, then the top footswitch 264 will, as suggested by the legend in field 84b, control the rapid-up command to the IV pole. In this regard, the top footswitch 264 will have the same function performed by membrane switch 62c shown in secondary front panel 60 on Figure 2.

Figure 17 shows the custom title setup screen 764, which is entered by depressing button 56b when the Figure 9 menu is displayed. This menu is used to allow a user to create a custom title to be displayed in the lower field of top region 92. The custom titles so created by use of this menu will preferably be displayed on every menu which is presented on display 50. In this menu a central field 810 is provided with a line 812 drawn thereon. A character selection area 814 is also provided with the available set of characters which can be used by the user to construct any desired custom title. Normally, the characters set provided in the character selection area or field 814 will correspond to the language in which the legends are currently displayed. Also numbers and punctuation marks and the like may be provided if desired as part of the character set.

Display portions 88a, 88c and 88e respectively control the position of the character cursor, the line cursor and the title cursor via button pairs 58a, 58c and 58e. The character cursor input switch pair 58 is used to move the character cursor, that is represented by the rectangle 20, to the letter to be selected. The up arrow 58a-1 will move the cursor to the left, and the down arrow 58-2 will move the cursor to the right. The line cursor input switch pair 58c moves the character cursor between the lines within field 814, with the up arrow moving the cursor up, and the down arrow moving the cursor down. The selected character is written to the title field 810 by pressing the title cursor down arrow 58e-2. The selected character will then appear on the title line 812. The title cursor up arrow when pressed moves the title cursor to the left on line 812 by one space.

The character cursor 820 is moved to the next desired letter via buttons 58a and 58c, and the newly selected characters written by depressing button 58e-2. This process is repeated by the user until the entire desired title is formed. Pressing the exit button 58g causes the title to be stored and used in a manner described above in display field 96.

Figure 18A shows the power-up mode setup screen 766, and is entered by pressing button 56a when the Figure 9 menu is displayed.- Screen 766 presents three options to the user shown in display fields 84a-84c, which are selected by corresponding buttons 54a-54c. If button 54a is depressed, the anterior tool utilities menu shown in Figure 18B will be presented. When button 54b is selected, the posterior tool setup menu 774 shown in Figure 18C will be displayed on display 50. Pressing button 54c when in menu 766 causes the main menu to be presented on screen 50 when the console 46 is first powered up. As will now be explained, it is possible for the user, via menus 764, 772 and 774 shown in Figures 18A, 18B and 18C to have different menus displayed upon power up if so desired.

Figure 18B shows the anterior tool setup menu 772, which is entered by depressing 54a when the Figure 18A menu is displayed. This menu 772 presents six choices in display fields 84a-84f and 86a. Any one of the screens listed in these fields may be caused to appear first when the console 46 is powered up by selecting them via buttons 54a-54f or 56a.

Figure 18C shows menu 774, which in a manner like that of Figure 18B, allows the user to select which of the six posterior screens listed In fields 84a-84f the user may wish to see first upon powering up the console 46. Like the other screens, the user's choice will be saved when the last-used power-up utility screen 766, 772 or 774 is exited by pushing button 58g.

VI. Epilogue

As may be seen from the aforegoing description presented above, the utilities menus shown in Figures 9-18 provide a user with a tremendous ability to customize the control console 46 to his or her liking. Further, as explained, the change of rise times in aspiration levels allows the system 40 to simulate the operational characteristics of other microsurgical equipment. Finally, the ability to generate tones as desired to shut them off, allows the surgeon to adapt the control console 46 to his or her personal desires.

The foregoing detailed description shows that the preferred embodiments of the present invention are well suited to fulfill the objects above-stated. It is recognized that those skilled in the art may make various modifications or additions to the preferred embodiments chosen to illustrate the present invention without departing from the spirit and proper scope of the invention. For example, the order or titles of the legends may be changed, and the layouts of the screens may be varied. Also, different arrangements for or types of switches or buttons may be utilized. Accordingly, it is to be understood that the protection sought and to be afforded hereby should be deemed to extend to the subject matter defined by the appended claims, including all fair equivalents thereof.

**Claims**

1. An ophthalmic microsurgical control system for controlling a plurality of micro surgical instruments, the control system comprising:
a main processor;

display means;

a plurality of operator-actuatable input switches;

means for generating a plurality of set-up menus on said display means, each of said menus listing one or more parameters which may be adjusted; and

means for adjusting at least one of the parameters on at least a plurality of the available set-up menus.

2. The control system of Claim 1, wherein one of the set-up menus relates to adjustment of audio tones; and

means for providing a plurality of different audio tones.

3. The control system of claim 1, further comprising:

means for controlling an aspiration function used in connection at least one of the surgical instruments; and

means for adjusting a rate at which an aspiration level is allowed to change.

4. The control system of claim 3, wherein at least one of the set-up menus relates to adjustments of the rate at which the aspiration is allowed to change from a first level to a second level.

5. The control system of claim 1, wherein at least one set-up menu lists at least three different user-selectable rates at which the aspiration is allowed to change.

6. The control system of claim 5, wherein the means for adjusting the rate of change of the aspiration level includes means for limiting the rate at which a command input signal changes.

7. The control system of claim 6, wherein the maximum rate at which the command signal may rise is user-selectable within a predetermined range of rates.

8. A microsurgical control system for controlling a plurality of microsurgical instruments, said system comprising:

a main control console;

a main processor;

display means for simultaneously displaying a plurality of fields of information pertaining to controlling at least one of the microsurgical instruments;

operator input means for adjusting control parameters associated with the instruments;

audio generation means for programmably producing a plurality of different sounds; and

means for providing a plurality of choices of different sounds for different surgical functions via one of said display means.

9. The control system of claim 8, wherein the different sounds include different tones.

10. The control system of claim 8, wherein the audio generation means produces sounds periodically at predetermined tones, said sounds including at least first and second periodic presentation rates.

11. The control system of claim 8, wherein the different sounds include tones of at least three different frequencies.

12. The control system of claim 8, wherein:

the means for providing produces at least one display screen with at least first, second and third regions containing different types of information relating to adjustments of generated sounds;

the first display region listing at least a plurality of different surgical procedures,

the second display region listing a plurality of selections of different tones, and

a third region for providing a legend relating to adjusting the volume of at least one tone.

13. The control system of claim 8, wherein the operator input means includes a plurality of operator input switches, and the means for providing includes a display screen for adjusting a parameter setting related to a volume of at least one sound, with the first one of the input switches being for increasing the volume of the sound, and the second one of the switches being for decreasing the volume of the sound.

14. The control system of claim 8, further comprising non-volatile memory means for permanently retaining user-entered choices relating to sounds.

15. An ophthalmic microsurgical control console for operating a plurality of microsurgical instruments, comprising:

display means for providing a plurality of user-selectable menus to be visually presented;

microprocessor means for producing set-up menus arranged in a predetermined organizational structure;

operator input means for selecting any one of the set-up menus; and

non-volatile memory means for storing user-selected parameter values.

16. The control console of claim 15, further comprising:

means for adjusting default parameters associated with a plurality of anterior segment surgical procedures; and

means for adjusting default parameters associated with posterior segment surgical procedures.

17. The control console of claim 16, wherein the operator input means includes means for causing a recording of user-selected parameters in the non-volatile memory means.

FIG.IA

FIG.IB

FIG.2

FIG.3

FIG. 4A.

*FIG. 4B.*

*FIG. 4C.*

24

...

FIG. 4 D.

FIG. 4 E.

FIG. 4 F.

EP 0 424 686 A1

FIG. 5

27

FIG.6

EP 0 424 686 A1

FIG. 7A

ANTERIOR SEGMENT

CAC

RESET TIME

IRR PRIME

ASP PRIME

PHACO CAL

MAX ASP
30
mmHg

88e
88e-1
88e-3

IRRIGATION
IRR/ASP
PHACO
VITRECTOMY
BIPOLAR
IOP CONTROL

84b
54b
82
724

RISE TIME:1
ASP
780a
780b

0
ACTUAL
mmHg

INFO ?

◄ ►  ◄ ►  ◄ ►  ◄ ►  ◄ ►

EXIT ⚠

58e-1
58e-2

FIG. 7B

FIG. 7C

EP 0 424 686 A1

## ANTERIOR SEGMENT

IRRIGATION

IRR /ASP

PHACO

VITRECTOMY

BIPOLAR

IOP CONTROL

CAC

RESET TIME

IRR TIME

ASP PRIME

PHACO CAL

LINEAR PHACO

| 0 % | 0:00 |
|---|---|

AVERAGE POWER     ELASPED TIME

OFF IRR ASP PHACO

RISE TIME:1    0 ACTUAL
ASP          mmHg

| MIN PHACO | MAX PHACO | PULSE RATE | FIXED ASP |
|---|---|---|---|
| 5 | 5 | 0 | 30 |
| % | % | PPS | mmHg |

728

INFO ?

▲ ▲ ▲ ▲ ▲

▼ ▼ ▼ ▼ ▼

EXIT ⚠

## FIG. 7D

EP 0 424 686 A1

*FIG. 8A*

POSTERIOR SEGMENT

VITRECTOMY

FRAGMENTATION

SCISSORS

BIPOLAR

ILLUMINATION

IOP CONTROL

RESET TIME

ASP PRIME

FRAG CAL

INFO ?

EXIT

EP 0 424 686 A1

POSTERIOR SEGMENT ⟍736

VITRECTOMY

FRAGMENTATION

SCISSORS

BIPOLAR

794 → IOP ⟍794a | O ACTUAL mm Hg ⟍794b

84e
ILLUMINATION

RESET TIME

780 782 86e
RISE TIME : 1

84f ASP | O ACTUAL mm Hg

ASP PRIME

FRAG CAL

734

IOP CONTROL

736 PRESSURE 20 mm Hg
88a

INTENSITY 100 %

734 88b

INFO ?

▲ ▲ ▲ ▲ ▲

▼ ▼ ▼ ▼ ▼

EXIT ⚠

FIG. 8B

EP 0 424 686 A1

FIG. 8C

UTILITIES MENU

54a

ANT SETTINGS ～84a                    86a ～ POWER-UP MODE

54b
POST SETTINGS                              CUSTOM TITLE

ASPIRATION                                   LANGUAGES ～ 56c

VITRECTOMY                    86d ～ AUDIO CONTROL — 56d

ACCESSORY ～84e

                                    86f --- ⌐SAVE⌐

718

INFO ⌐?⌐        ▲    ▲    ▲    ▲    ▲        EXIT ⚠

                ▼    ▼    ▼    ▼    ▼

*FIG.9*

EP 0 424 686 A1

ANTERIOR INPUT SETTING

IRRIGATION                                          CAC

54b — IRR/ASP

54c — PHACO

54d — VITRECTOMY

54e — BIPOLAR

54f — IOP CONTROL          88a

742

| | BIPOLAR | | | |
|---|---|---|---|---|
| | 0 | | | |
| | % | | | |

88e-2

88b                                    88e

INFO ?    ▲  ▲  ▲  ▲  ▲    EXIT ⚠

▼  ▼  ▼  ▼  ▼

58b    58c    58e

FIG. 10

POSTERIOR INPUT SETTINGS

VITRECTOMY

FRAGMENTATION

SCISSORS

BIPOLAR

ILLUMINATION

IOP CONTROL

744

INFO ?

▲ ▲ ▲ ▲ ▲

▼ ▼ ▼ ▼ ▼

EXIT ⚠

*FIG. 11*

EP 0 424 686 A1

EP 0 424 686 A1

ASPIRATION SETUP

**54**

1 SEC RISE TIME ~*84a*

2 SEC RISE TIME

3 SEC RISE TIME

4 SEC RISE TIME

5 SEC RISE TIME

6 SEC RISE TIME ~*84f*

**746**

INFO ?  ▲ ▼  ▲ ▼  ▲ ▼  ▲ ▼  ▲ ▼  EXIT ⚠

*FIG. 12*

EP 0 424 686 A1

VITRECTOMY SETUP

STORZ VIT ~ *84a*

MICROVIT VIT

*748*

INFO ?

▲ ▲ ▲ ▲ ▲

▼ ▼ ▼ ▼ ▼

EXIT ⚠

*FIG. 13*

LANGUAGE SETUP

ENGLISH

DEUTSCH

ESPANOL

FRANCAIS

ITALIANO

750

INFO ?

EXIT ⚠

*FIG. 14*

AUDIO CONTROL SETUP

54a

ASPIRATION ~84a

PHACO/FRAG TONE

BIPOLAR TONE

WARNING TONES

54b

54d

752

TONE OFF

TONE 1

TONE 2

TONE 3

TONE 4

TONE 5

56a

56f

88c

VOLUME ADJ

050

%

INFO ?

▲

▲

▲

▲

▲

▼

▼

▼

▼

▼

58c

EXIT ⚠

EP 0 424 686 A1

42

FIG. 15

ACCESSORY SETUP

54a

BACK PANEL

54b

IV POLE RAPID UP — 84b

762

INFO ?  ▲ ▼  ▲ ▼  ▲ ▼  ▲ ▼  ▲ ▼  EXIT ⚠

FIG. 16

EP 0 424 686 A1

CUSTOM TITLE SETUP

A

810
812
824

820

A B C CHARACTER SELECTION J K L M
N O P Q AREA W X Y Z
1 2 3 4 . . . . . . , * ! ?

814

CHAR CURSOR
88a

LINE CURSOR
88c

TITLE CURSOR
88e

764

INFO ?

58a-1
58a

58c

58e-1
58e
58e-2

EXIT

FIG. 17

POWER-UP MODE SETUP

ANTERIOR — 84a

POSTERIOR

MAIN MENU — 84c

766

INFO ?    ▲    ▲    ▲    ▲    ▲    EXIT ⚠
          ▼    ▼    ▼    ▼    ▼

## FIG. 18A

ANTERIOR TOOL SETUP

IRRIGATION — 84a

86a — CAC

IRR/ASP

PHACO

VITRECTOMY

BIPOLAR

IOP CONTROL — 84f

772

INFO [?]

EXIT ⚠

FIG. 18B

EP 0 424 686 A1

POSTERIOR TOOL SETUP

VITRECTOMY ~84a
FRAGMENTATION
SCISSORS
BIPOLAR
ILLUMINATION
IOP CONTROL ~84f

774

58g
EXIT ⚠

INFO ?

FIG. 18C

47

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | US-A-4 773 897 (STORZ) <br> * Column 3, line 65 - column 4, line 1 * <br> — — — | 1-17 | A 61 B 17/32 <br> A 61 F 9/00 |
| P,Y | DE-A-3 923 024 (SIEMENS) <br> * Claim 1 * <br> — — — | 1-17 | |
| A | US-A-3 961 630 (GONSER) <br> * Column 1, lines 22-33 * <br> — — — | 2,8,9 | |
| A | GB-A-2 132 893 (MIODUSKI) <br> * Abstract * <br> — — — | 2,8-10 | |
| A | US-A-4 770 654 (ROGERS et al.) <br> — — — — — | | |

| | TECHNICAL FIELDS SEARCHED (Int. Cl.5) |
|---|---|
| | A 61 B <br> A 61 F <br> A 61 M |

The present search report has been drawn up for all claims

| Place of search | Date of completion of search | Examiner |
|---|---|---|
| The Hague | 30 January 91 | GLAS J. |